# EUROPEAN PATENT APPLICATION

(11) **EP 4 104 768 A1**
(43) Date of publication of application: **21.12.2022**
(21) Application number: 20918681.6
(22) Date of filing: 14.02.2020
(51) Int. Cl.: A61B 8/00

(54) **ULTRASOUND PROBE OPERATION SYSTEM AND METHOD**

(71) Applicant: Asahi Intecc Co., Ltd., Seto-shi, Aichi 489-0071 (JP); Fuji Corporation, Chiryu-shi, Aichi 472-8686 (JP)
(72) Inventor: YOSHIDA, Naofumi, Chiryu-shi, Aichi 472-8686 (JP); YAMASHITA, Yasuhiro, Chiryu-shi, Aichi 472-8686 (JP); HIRANO, Keisuke, Yokohama-shi, Kanagawa 227-0067 (JP); SAITO, Hiroyuki, Yokosuka-shi, Kanagawa 237-0067 (JP); NISHIUCHI, Makoto, Seto-shi, Aichi 489-0071 (JP); SHIMOGAMI, Manabu, Seto-shi, Aichi 489-0071 (JP); OSHIMA, Fumiyoshi, Seto-shi, Aichi 489-0071 (JP)
(74) Representative: Winter, Brandl - Partnerschaft mbB
(86) International application number: PCT/JP2020/005822
(87) International publication number: WO 2021/161516

(57) **Abstract**

To provide an ultrasound probe operation system and method that can improve usability for the user. This system is for operating an ultrasound probe 111 and is provided with: sensor operation portions 11, 12 for operating the ultrasound probe that acquires ultrasound echo images 21, 22 of a blood vessel 4; and a control portion 1 that controls operations of the sensor operation portions. The control portion may switch the arrangement of the ultrasound probe using the sensor operation portions between a first arrangement for acquiring an image of a cross section in the transverse direction of the blood vessel and a second arrangement for acquiring an image of a cross section in the longitudinal direction of the blood vessel.

## Description

### TECHNICAL FIELD

The disclosed embodiments relate to an ultrasound probe operation system and relate also to a method thereof.

### BACKGROUND ART

In treatment using a catheter, a first operator (a doctor, an operator, or the like) presses an ultrasound probe against a body surface of a patient to acquire ultrasound echo images of a blood vessel and a guide wire in the blood vessel, and a second operator (a doctor or a technician) inserts the guide wire into the blood vessel while confirming the blood vessel and a position of the guide wire by checking the ultrasound echo image. The position of the guide wire to be confirmed includes a position of a distal end of the guide wire in a blood vessel axial direction and a position of a distal end of the guide wire in a cross section in the blood vessel.

To properly and promptly send the guide wire to a predetermined position in the blood vessel, it is necessary to coordinate an operation of the ultrasound probe and an operation of the guide wire, however, it is difficult for the first operator and the second operator to work in harmony to execute a subtle operation and a burden to each operator is large.

In a conventional technology, there is known a method for applying an index for arranging the ultrasound probe to intersect with a central axis of the blood vessel, for example, an index such as a change in blood vessel cross section area in synchronization with a heartbeat (Patent Literature 1).

### CITATION LIST

### Patent Literature

Patent Literature 1: Japanese Unexamined Patent Application Publication No. 2004-229823

### SUMMARY OF INVENTION

### TECHNICAL PROBLEM

In the conventional technology, which is a technology only for outputting an index for the arrangement of the ultrasound probe, it is difficult to accurately detect a cross section in the longitudinal direction and a cross section in the transverse direction of the blood vessel, so that user-friendliness is low for a doctor, an operator, or the like. In the conventional technology, it is also difficult to identify the position of the distal end of the guide wire in the blood vessel at almost the same time in both a cross section in the longitudinal direction and a cross section in the transverse direction of the blood vessel.

The disclosed embodiments are made in view of the above problems, and an object of the disclosed embodiments is to provide an ultrasound probe operation system with improved user-friendliness and a method thereof.

### SOLUTION TO PROBLEM

To solve the above problems, an ultrasound probe operation system according to one aspect of the disclosed embodiments is an ultrasound probe operation system for operating an ultrasound probe, the system including a sensor operation portion that operates an ultrasound probe that acquires an ultrasound echo image of a blood vessel and a control portion that controls an operation of the sensor operation portion. The control portion may switch an arrangement of the ultrasound probe using the sensor operation portion between a first arrangement for acquiring an image of a cross section in a transverse direction of the blood vessel and a second arrangement for acquiring an image of a cross section in a longitudinal direction of the blood vessel.

The operation portion may switch the arrangement between the second arrangement and the first arrangement by rotating the ultrasound probe around a predetermined rotation axis.

The ultrasound probe operation system further includes a display portion that displays the ultrasound echo image acquired by the ultrasound probe, and the control portion may display a first ultrasound echo image acquired in the first arrangement and a second ultrasound echo image acquired in the second arrangement in an associated manner on the display portion.

The control portion may display, on the display portion, an ultrasound echo image corresponding to a current or past arrangement of the ultrasound probe, separately from an ultrasound echo image corresponding to an arrangement other than the current or past arrangement, out of the first ultrasound echo image and the second ultrasound echo image.

The ultrasound echo image includes an image of a long medical device in the blood vessel, and the control portion may calculate an amount of deviation between the long medical device and the blood vessel in the first ultrasound echo image and/or the second ultrasound echo image.

The control portion may display the calculated amount of deviation on the display portion in association with the first ultrasound echo image and/or the second ultrasound echo image.

The control portion may output an alarm when the amount of deviation reaches a predetermined threshold value.

The control portion may detect center positions of the blood vessel in the first ultrasound echo image at a plurality of locations separated in the longitudinal direction of the blood vessel, and based on a distance between the center positions and a distance between the plurality of locations, calculate an inclination of the blood vessel in the longitudinal direction.

The control portion may set the ultrasound probe to the first arrangement at the plurality of locations separated in the longitudinal direction of the blood vessel to detect the center positions of the blood vessel in the first ultrasound echo image, and based on a distance between the center positions and a distance between the plurality of locations, calculate an inclination of the blood vessel in the longitudinal direction to correct the amount of deviation, based on the calculated inclination.

The control portion may acquire the second ultrasound echo image as a longitudinal cross section image passing through each of the center positions of the blood vessel, based on the calculated inclination of the blood vessel in the longitudinal direction.

The control portion may control a posture of the ultrasound probe according to the calculated inclination of the blood vessel in the longitudinal direction.

The control portion may press the ultrasound probe against a body surface by inclining the ultrasound probe according to the calculated inclination of the blood vessel in the longitudinal direction to set the ultrasound probe to the second arrangement so that an image of the blood vessel in the longitudinal direction is displayed horizontally in the second ultrasound echo image.

The control portion may switch the arrangement between the first arrangement and the second arrangement by rotating , by a predetermined angle, the inclined ultrasound probe being pressed against the body surface.

The control portion may detect center positions of the blood vessel in the first ultrasound echo image at a plurality of locations separated in the longitudinal direction of the blood vessel, and based on a distance between the center positions and a distance between the plurality of locations, detect a route of the blood vessel.

The disclosed embodiments may be grasped as an ultrasound probe operation method.

According to the disclosed embodiments, it is possible to switch an arrangement of an ultrasound probe between a first arrangement for acquiring an image of a cross section in the transverse direction of a blood vessel and a second arrangement for acquiring an image of a cross section in the longitudinal direction of the blood vessel, and thus, it is possible to improve user-friendliness.

### BRIEF DESCRIPTION OF DRAWINGS

FIG. 1 is an explanatory diagram illustrating an overview of the present embodiment.
FIG. 2 is a flowchart of a process according to the present embodiment.
FIG. 3 is an explanatory diagram illustrating an example of a configuration of an embodiment.
FIG. 4 is a basic diagram for explaining an operation of an ultrasound probe.
FIG. 5 is a top view, a front view, and a side view of the ultrasound probe.
FIG. 6 is an explanatory diagram illustrating a state in which a cross section of a blood vessel is detected by the ultrasound probe.
FIG. 7 is an explanatory diagram in continuation to FIG. 6.
FIG. 8 is an explanatory diagram in continuation to FIG. 7.
FIG. 9 is an explanatory diagram in continuation to FIG. 8.
FIG. 10 is an explanatory diagram in continuation to FIG. 9.
FIG. 11 is an explanatory diagram in continuation to FIG. 10.
FIG. 12 is a flowchart of a process for searching a blood vessel.
FIG. 13 is a flowchart illustrating a process for searching a longitudinal cross section of a blood vessel.
FIG. 14 is a flowchart of a process for searching a longitudinal cross section of a blood vessel according to a second embodiment.
FIG. 15 is a flowchart of a process for searching a longitudinal cross sectional view of a blood vessel according to a third embodiment.
FIG. 16 is a flowchart illustrating a process for operating an ultrasound probe and a guide wire according to a fourth embodiment.
FIG. 17 is a flowchart in continuation to FIG. 16.
FIG. 18 is a flowchart illustrating a blood vessel search process according to a fifth embodiment.
FIG. 19 is a flowchart in continuation to FIG. 18.
FIG. 20 is a flowchart illustrating a blood vessel search process according to a sixth embodiment.
FIG. 21 is a flowchart in continuation to FIG. 20.
FIG. 22 is an explanatory view illustrating a screen corresponding to an operation of an ultrasound probe and a guide wire according to a seventh embodiment.
FIG. 23 is an explanatory diagram in continuation to FIG. 22.
FIG. 24 is an explanatory diagram in continuation to FIG. 23.
FIG. 25 is an explanatory diagram in continuation to FIG. 24.
FIG. 26 is an explanatory diagram in continuation to FIG. 25.

### EMBODIMENTS OF THE INVENTION

Examples of a long medical device used in the present embodiment include a guide wire, a guiding catheter, a microcatheter, a balloon catheter, a cutting balloon, and a stent delivery device.

An embodiment of the disclosed embodiments will be described with reference to the drawings below. When an embodiment in which the long medical device is used in the disclosed embodiments is described, a case where the long medical device is a guide wire is used as an example. In the present embodiment, description proceeds based on a case where an ultrasound probe for an ultrasound diagnostic device is operated as an example, but the present invention may also be applied to an ultrasound probe used in a device other than the ultrasound diagnostic device.

In the present embodiment, the ultrasound probe is positioned differently in direction from a blood vessel to detect a position of the blood vessel. As an example, in the present invention, the ultrasound probe is switched in arrangement between a first arrangement for acquiring an image of a cross section in the transverse direction of the blood vessel and a second arrangement for acquiring an image of a cross section in the longitudinal direction of the blood vessel to detect the position of the blood vessel. The "cross section in the transverse direction of the blood vessel" means a cross section obtained when the blood vessel is cut along a plane intersecting a central axis of the blood vessel.

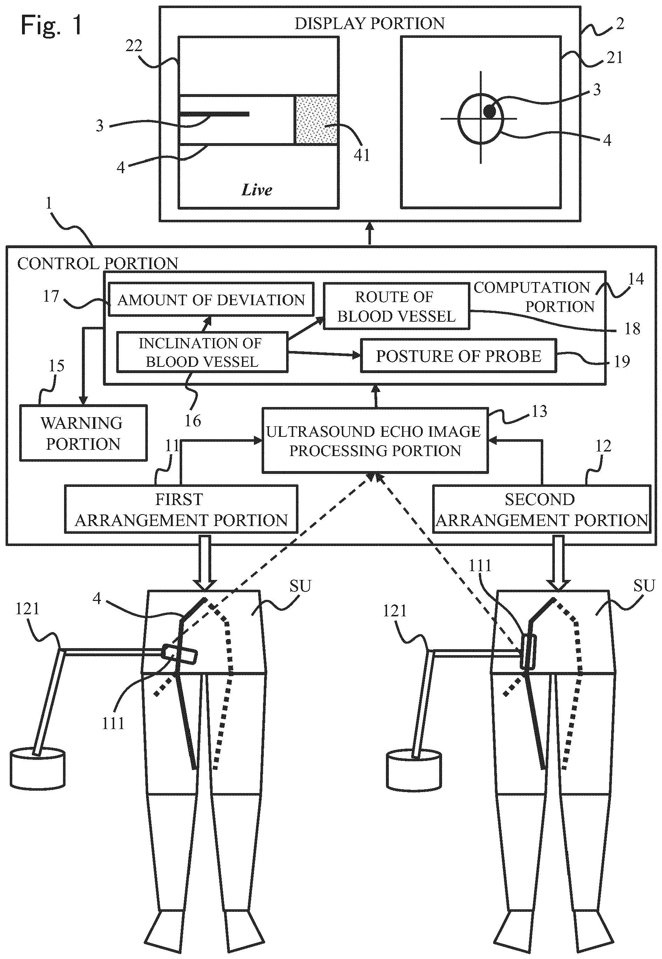

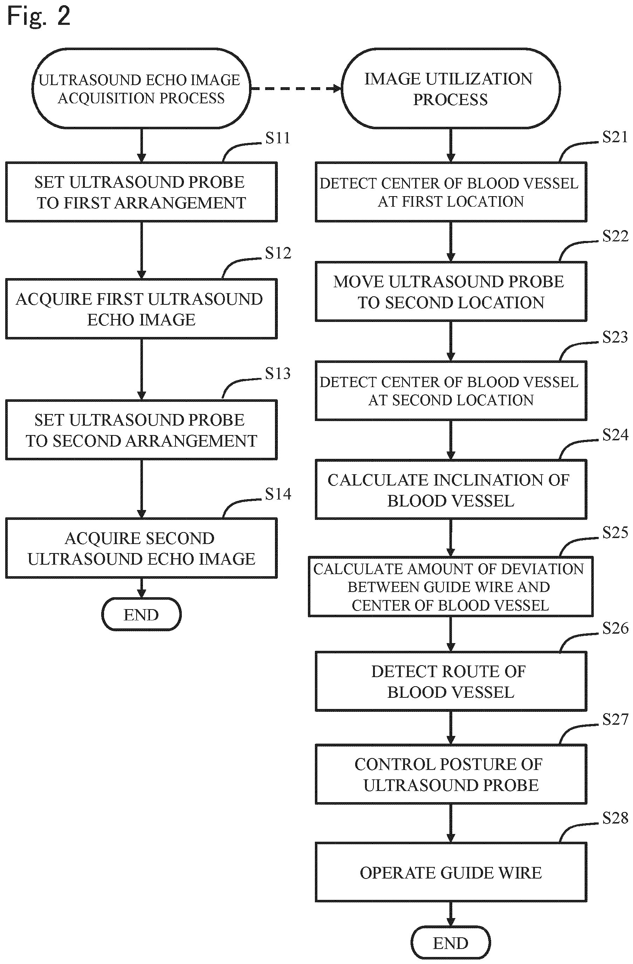

With reference to FIGS. 1 and 2, an overview of the present embodiment will be described. A plurality of embodiments included in the present embodiment will be described later with reference to another drawing. FIGS. 1 and 2 illustrate an overview of the present embodiment and do not define the scope of the disclosed embodiments. The disclosed embodiments may be configured from a part of the configurations disclosed in FIGS. 1 and 2, or the disclosed embodiments may be configured by including a configuration not disclosed in FIGS. 1 and 2.

An ultrasound probe operation system (hereinafter referred to as "probe operation system") according to the present embodiment includes a control portion 1, a display portion 2, an ultrasound probe 111, and a robot 121, for example. A first arrangement portion 11, a second arrangement portion 12, the robot 121, and a robot control device 120 described later correspond to an example of a "sensor operation portion".

For example, the ultrasound probe 111 is pivotably attached to a distal end of the robot 121 which is a six-axis robot. An image (ultrasound echo image) captured by the ultrasound probe 111 is sent to the control portion 1 for processing. In the following description, the ultrasound echo image may be abbreviated as an echo image or an image.

The control portion 1 includes the first arrangement portion 11, the second arrangement portion 12, an ultrasound echo image processing portion 13, a computation portion 14, and a warning portion 15, for example.

The first arrangement portion 11 includes a function of arranging the ultrasound probe 111 in a direction crossing a blood vessel 4 to capture an image of a cross section in the transverse direction of the blood vessel 4 of a subject SU. The second arrangement portion 12 includes a function of arranging the ultrasound probe 111 along a longitudinal direction of the blood vessel 4 to capture an image of a cross section in the longitudinal direction of the blood vessel 4.

Hereinafter, the cross section in the transverse direction of the blood vessel may be referred to as "first cross section" (transverse cross section or transverse section), and the cross section in the longitudinal direction of the blood vessel may be referred to as "second cross section" (longitudinal cross section or longitudinal section). The transverse direction of the blood vessel may be referred to as "short axis direction of the blood vessel", and the longitudinal direction of the blood vessel may be referred to "long axis direction of the blood vessel".

The ultrasound echo image processing portion 13 includes a function of acquiring a first ultrasound echo image from the ultrasound probe 111 placed in the first arrangement by the first arrangement portion 11 and a second ultrasound echo image from the ultrasound probe 111 placed in the second arrangement by the second arrangement portion 12. In the following description, the first ultrasound echo image may be referred to as "image of the first cross section", and the second ultrasound echo image may be referred to as "image of the second cross section".

The computation portion 14 includes a function of analyzing the first ultrasound echo image and the second ultrasound echo image acquired from the ultrasound echo image processing portion 13. The computation portion 14 includes a function 16 of calculating the inclination of the blood vessel, a function 17 of calculating the amount of deviation, a function 18 of detecting the route of the blood vessel, and a function 19 of controlling the posture of the ultrasound probe 111, for example.

The function 16 of calculating the inclination of the blood vessel calculates an inclination of the blood vessel 4. For example, the function 16 of calculating the inclination of the blood vessel may detect each of center positions of the blood vessel in the first ultrasound echo image at a plurality of locations separated in the longitudinal direction of the blood vessel 4, and based on a distance between the center positions and a distance between the plurality of locations, calculate an inclination of the blood vessel in the longitudinal direction. It is possible to calculate the inclination of the blood vessel, based on the center position of the blood vessel detected first, from a difference from a center position of the blood vessel detected next and a distance between the center positions of the blood vessel. Instead thereof, when a three-dimensional space in which the ultrasound probe is operated is firstly defined, it is possible to evaluate an inclination (direction) of a line linking two different center positions of the blood vessel according to a vector calculation, from coordinates of the two center positions of the blood vessel in the three-dimensional space. In such a case, the distance between the center positions of the blood vessel may be unknown.

The deviation amount calculation function 17 calculates an amount of deviation (distance) between the center of the transverse section of the blood vessel 4 and the distal end of the guide wire 3, based on the calculated inclination of the blood vessel.

The function 18 of detecting the route of the blood vessel detects the route of the blood vessel 4, based on the calculated inclination of the blood vessel. The function 18 of detecting the route of the blood vessel may detect the center positions of the blood vessel in the first ultrasound echo image at a plurality of locations separated in the longitudinal direction of the blood vessel 4, and based on a distance between the center positions and a distance between the plurality of locations, detect a route of the blood vessel 4.

The function 19 of controlling the posture of the ultrasound probe is a function of causing the posture of the ultrasound probe 111 to follow according to the inclination and the route of the blood vessel, based on the calculated inclination of the blood vessel 4.

The probe posture control function 19 presses the ultrasound probe 111 against a body surface by inclining the ultrasound probe 111 according to the calculated inclination of the blood vessel in the longitudinal direction to set the ultrasound probe 111 to the second arrangement so that an image of the blood vessel in the longitudinal direction is displayed horizontally in the second ultrasound echo image 22. It is noted that the image 22 may be processed so that instead of the posture of the ultrasound probe 111 being changed, the blood vessel 4 in the second ultrasound echo image 22 is displayed horizontally.

Arrows connecting the functions 16 to 19 in the computation portion 14 are an example. A relationship among the functions 16 to 19 is not limited to the arrows illustrated in FIG. 1 or the direction of the arrows.

The warning portion 15 is a function of issuing a warning to a user if an arithmetic operation result by the computation portion 14 reaches a predetermined threshold value. For example, if the amount of deviation calculated by the deviation amount calculation function 17 reaches a predetermined threshold value, the warning portion 15 warns the user by an alarm, a warning message, or a combination thereof. The warning portion 15 may warn the user not only for the amount of deviation but also, for example, the inclination of the blood vessel, the route of the blood vessel, and the posture of the ultrasound probe 111. The predetermined threshold value may be arbitrarily set by the user, or may be a value calculated from history data of a surgery.

The display portion 2 presents the ultrasound echo image to the user. The display portion 2 provides such an image to the user by displaying one or both of the first ultrasound echo image 21 and the second ultrasound echo image 22 on the screen. The display portion 2 may directly display a raw image from the ultrasound probe 111 on the screen or may display a result obtained by applying image processing such as a contour enhancement on the raw image on the screen.

The display portion 2 displays such an image to associate the first ultrasound echo image 21 (first cross section image) and the second ultrasound echo image 22 (second cross section image) (so that, for example, the blood vessel 4 displayed in the image 21 and the blood vessel 4 displayed in the image 22 are arranged in a predetermined positional relationship).

In FIG. 1, the image 21 and the image 22 are arranged side by side along a left-right direction of the display portion 2. The image 22 may be processed so that the blood vessel 4 in the second ultrasound echo image 22 is displayed horizontally. The image 22 may be processed so that an acute angle formed by a length direction of the blood vessel 4 in the second ultrasound echo image 22 and the left-right direction of the image 22 is smaller, or the both coincide in direction with each other.

The image 21 and the image 22 may be arranged side by side along an up-down direction of the display portion 2. The image 22 may be processed so that the blood vessel 4 in the image 22 is displayed vertically. The image 22 may be processed so that an acute angle formed by a length direction of the blood vessel 4 in the second ultrasound echo image 22 and the up-down direction of the image 22 is smaller, or the both coincide in direction with each other.

The display portion 2 displays a marker for distinguishing an image corresponding to a current arrangement of the ultrasound probe 111, out of the first ultrasound echo image 21 and the second ultrasound echo image 22. As an example, in FIG. 1, a word "Live" is used as the marker. In addition to a character such as words, a color, a screen size, or the like may be used as the marker. For example, an image corresponding to the current arrangement of the ultrasound probe 111 may be distinguished by enclosing such an image in red or the like. Alternatively, the image corresponding to the current arrangement of the ultrasound probe 111 may be distinguished by displaying such an image larger than the other images. Similarly, the display portion 2 may display the marker for distinguishing the image corresponding to a past arrangement of the ultrasound probe 111, out of the first ultrasound echo image 21 and the second ultrasound echo image 22.

In the present embodiment, as described later, the second arrangement and the first arrangement are switched by rotating the ultrasound probe around a predetermined rotation axis. The predetermined rotation axis is an axis used for pivoting the ultrasound probe 111 between the first arrangement and the second arrangement while a lens center point CP at the distal end of the ultrasound probe 111 is fixed at the current position. Such an accurate operation is realized by using the robot 121. However, simply by introducing the robot 121 into the ultrasound diagnostic device, it is not possible to reach a configuration in which the ultrasound probe 111 is pivoted between the first arrangement and the second arrangement without the movement of the center point CP.

With reference to a flowchart in FIG. 2, an example of a process executed by the probe operation system according to the present embodiment will be described. The left side in FIG. 2 illustrates a process for acquiring an ultrasound echo image by switching the arrangement of the ultrasound probe 111, and the right side in FIG. 2 illustrates a process for utilizing the acquired ultrasound echo image to operate the guide wire 3.

In the ultrasound echo image acquisition process, the control portion 1 sets the ultrasound probe 111 to the first arrangement (S11) and acquires the first ultrasound echo image (S12). The control portion 11 switches the ultrasound probe 111 from the first arrangement to the second arrangement (S13) to acquire the second ultrasound echo image (S14).

In the image utilization process, based on the first ultrasound echo image and the second ultrasound echo image, the control portion 1 detects the center in the first cross section (transverse section) of the blood vessel 4 at a first location, out of two locations along the longitudinal direction of the blood vessel (S21). The control portion 1 moves the ultrasound probe 111 in the longitudinal direction of the blood vessel 4 so that the ultrasound probe 11 is positioned at a point at a second location (S22). The control portion 1 detects the center in the first cross section of the blood vessel 4 at the second location (S23). When the blood vessel in the ultrasound echo image captured by the ultrasound probe 111 is tracked, it is possible to move the ultrasound probe 111 along the longitudinal direction of the blood vessel

The control portion 1 calculates the inclination of the blood vessel 4, based on the center of the blood vessel at the first location and the center of the blood vessel at the second point, and a movement distance of the ultrasound probe 111 from the first location to the second location (S24). As described above, based on the position of the center of the blood vessel at the first location, the position of the center of the blood vessel at the second location is relatively evaluated, and from the positions of the centers of the blood vessel and the distance between the first location and the second location, it is possible to calculate the inclination of the blood vessel. Instead thereof, in a defined three-dimensional space, the inclination of the blood vessel may be evaluated according to an arithmetic operation from coordinates of the center of the blood vessel at the first location and coordinates of the center of the blood vessel at the second location.

The control portion 1 calculates an amount of deviation between the position of the distal end of the guide wire 3 and the center in the first cross section of the blood vessel 4 (S25).

The control portion 1 detects the route of the blood vessel 4 (in which direction the blood vessel extends) by analyzing each ultrasound echo image (S26).

The user or the control portion 1 may control the posture of the ultrasound probe 111 to follow the blood vessel according to the route and the inclination of the blood vessel (S27).

The user or the control portion 1 operates the guide wire 3, based on information obtained in steps S21 to S27 (S28).

According to the present embodiment configured thus, it is possible to accurately detect the blood vessel to improve user-friendliness.

### [First Embodiment]

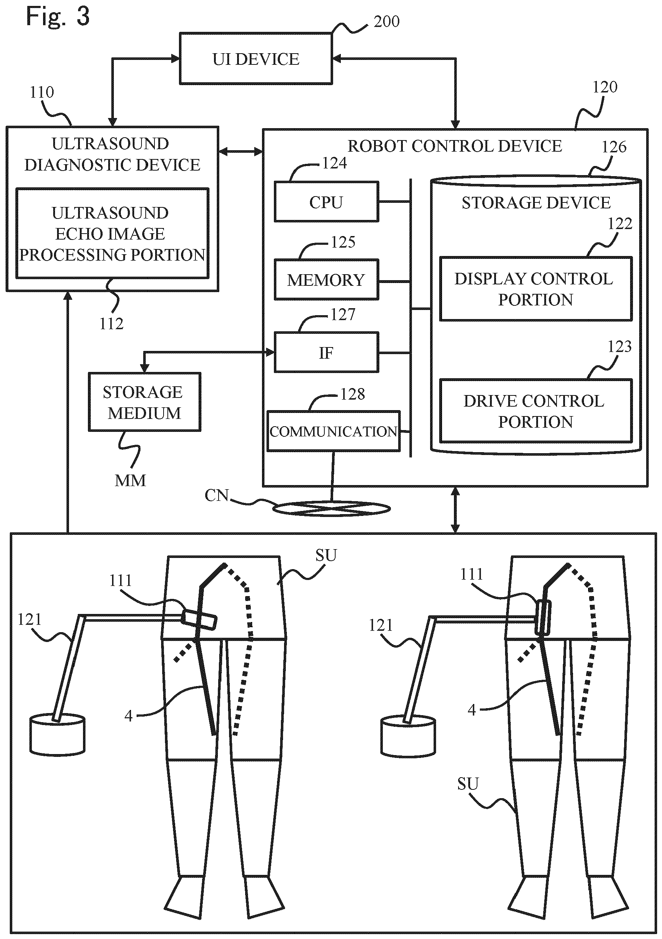

With reference to FIGS. 3 to 13, a first embodiment will be described. FIG. 3 is an explanatory diagram illustrating an example of a configuration of an ultrasound probe operation system for an ultrasound diagnostic device.

The probe operation system of the present embodiment includes an ultrasound diagnostic device 110, a robot control device 120, a robot 121, and a user interface (in FIG. 3, UI) device 200, for example.

The ultrasound diagnostic device 110 is an device that makes a diagnosis, based on the ultrasound echo image captured by the ultrasound probe 111. The ultrasound diagnostic device 110 includes an ultrasound echo image processing portion 112 that processes an ultrasound echo image.

The robot control device 120 controls the robot 121 configured as, for example, a six-axis robot. The robot control device 120 may also output a signal for controlling image capturing to the ultrasound diagnostic device 110.

The robot control device 120 includes a microprocessor (in FIG. 3, CPU: central processing unit) 124, a memory 125, a storage device 126, a medium interface 127, and a communication portion 128, for example. The robot control device 120 may be a dedicated device provided with a dedicated circuit, or may be a general-purpose computer for executing a predetermined computer program. The robot control device 120 may be linked with a plurality of devices. For example, one or more robot control devices 120 may be generated by coordinating a plurality of computers.

The storage device 126 stores therein a computer program for realizing the display control portion 122, another computer program for realizing a drive control portion 123, an operating system (not illustrated), and the like, for example.

As a result of causing predetermined computer programs 122 and 123 stored in the storage device 126 to be read and executed into the memory 125, the microprocessor 124 realizes each function as the probe operation system.

The medium interface 127 is a circuit that performs data communication with a storage medium MM such as a semiconductor memory or a hard disk. It is possible to store at least a part of the predetermined computer programs 122 and 123 in the storage medium MM and install the stored computer programs into the storage device 126 from the storage medium MM. Alternatively, it is possible to transfer at least a part of the predetermined computer programs 122 and 123 stored in the storage device 126 to the storage medium MM and store therein such programs. Instead of the storage medium MM, it is possible to use the communication network CN connected to the communication portion 128 of the robot control device 120 as a transmission medium for predetermined computer programs.

The user interface device 200 exchanges information between the ultrasound diagnostic device 110 and the robot control device 120. The user interface device 200 includes an information input device and an information output device. The information input device and the information output device may be integrated. Examples of the information input device include a keyboard, a push button, a voice input device, a touch panel, and a pointing device such as a mouse. Examples of the information output device include a monitor display, a printer, a voice synthesizer, and a light.

The lower left of FIG. 3 illustrates a case where the ultrasound probe 111 is set to the first arrangement, and the lower right of FIG. 3 illustrates a case where the ultrasound probe 111 is set to the second arrangement, respectively.

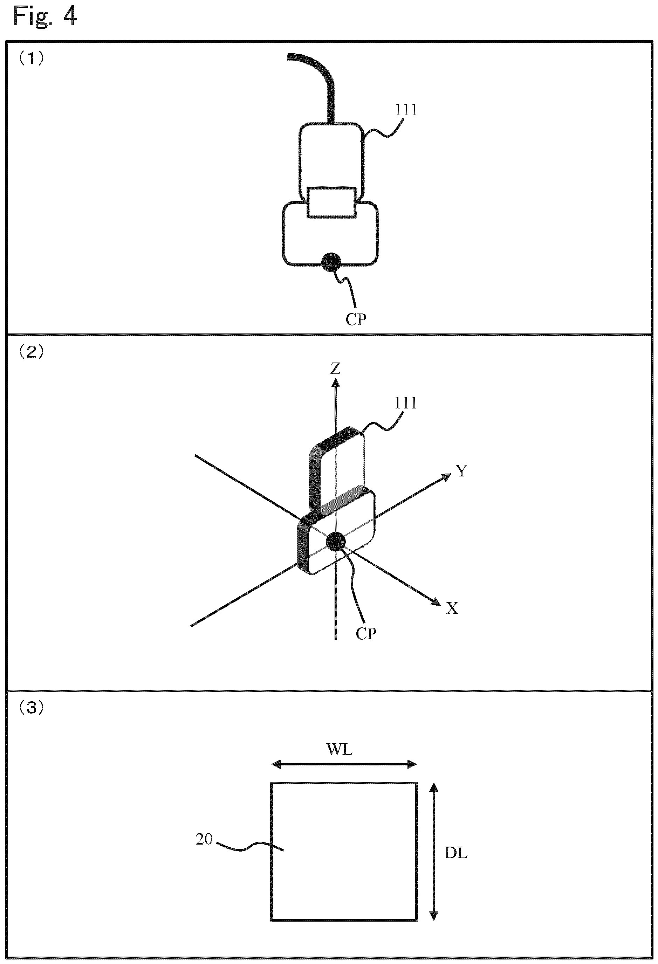

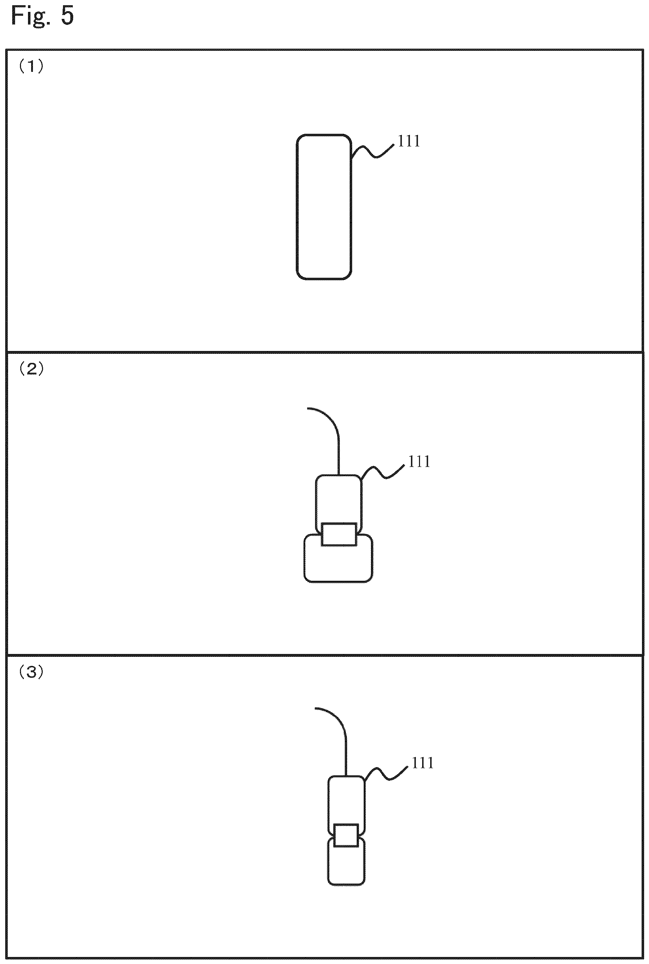

With reference to FIGS. 4 and 5, each portion of the ultrasound probe 111 will be defined to describe the operation of the ultrasound probe 111.

FIG. 4(1) is a view obtained when the ultrasound probe 111 is viewed from front where a center point CP is set to a center portion of a lens at the distal end of the ultrasound probe 111. In the present embodiment, the ultrasound probe 111 is switched between the first arrangement and the second arrangement while the center point CP is fixed.

FIG. 4(2) is a perspective view of the ultrasound probe 111. A central axis in a short axis direction of the ultrasound probe 111 is referred to as "X axis", a central axis in a long axis direction of the ultrasound probe 111 is referred to as "Y axis", and a central axis in a depth direction of the ultrasound probe 111 is referred to as "Z axis", respectively.

FIG. 4(3) illustrates an ultrasound echo image 20 captured by the ultrasound probe 111, where a horizontal direction of the image 20 is referred to as "width direction" of the image 20 and a vertical direction of the image 20 is referred to as "depth direction" of the image 20, respectively. The image 20 has a concept including the first ultrasound echo image 21 and the second ultrasound echo image 22.

FIG. 5(1) is a view obtained when the ultrasound probe 111 is viewed from above. FIG. 5(2) is a view obtained when the ultrasound probe 111 is viewed from front. FIG. 5(3) is a view obtained when the ultrasound probe 111 is viewed laterally.

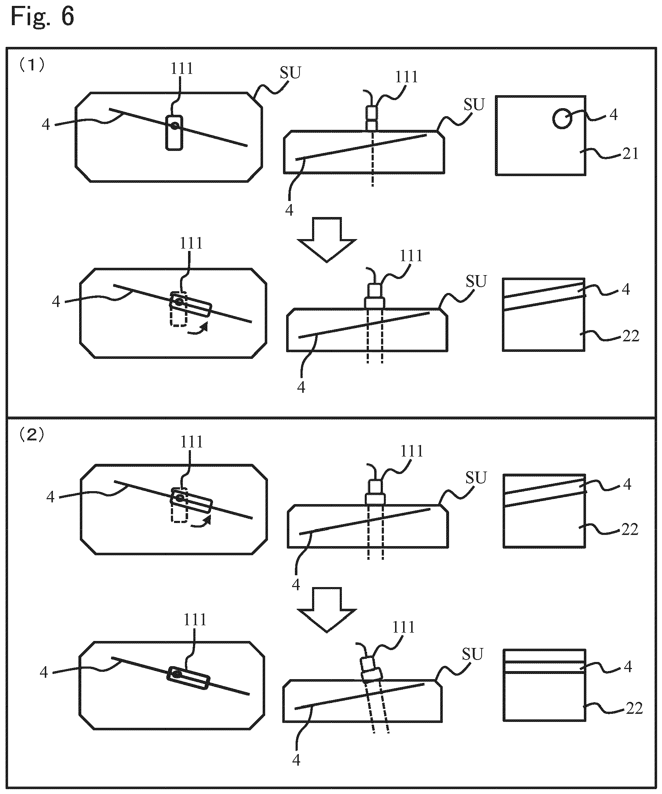

With reference to FIGS. 6 to 11, a state is described where the cross section (a transverse cross section and a longitudinal cross section) of the blood vessel 4 is detected by operating the ultrasound probe 111.

FIGS. 6 to 11 disclose a predetermined technology for evaluating a center of the cross section of the blood vessel 4 from the echo image 21 in the transverse direction of the blood vessel 4, recognizing the distal end portion of the guide wire 3 from such evaluation, and evaluating the amount of deviation of the guide wire 3 from the central axis of the blood vessel 4.

The predetermined technology includes detecting the center of the cross section of the blood vessel 4 from the echo image 21 in the transverse direction of the blood vessel 4 (transverse section of the blood vessel 4), and outputting the amount of deviation between the distal end portion of the guide wire and the center of the blood vessel 4.

The predetermined technology includes detecting the center of the cross section of the blood vessel 4 from the echo image 21 in the transverse direction of the blood vessel 4 to recognize the distal end portion of the guide wire 3, and issuing a warning if the amount of deviation between the guide wire 3 and the central axis of the blood vessel 4 exceeds a predetermined threshold value.

The predetermined technology includes calculating the inclination of the blood vessel 4 in the longitudinal direction from the center of the cross section in the transverse direction of the blood vessel 4 at a plurality of (two) locations and the movement amount of the ultrasound probe 111, and based on the calculated inclination of the blood vessel 4 in the longitudinal direction, correcting the amount of deviation of the guide wire 3 from the center of the blood vessel. That is, in such a case, after evaluating the center of the cross section of the blood vessel 4 from the echo image 21 in the transverse direction of the blood vessel 4, the ultrasound probe 111 is moved to a distant place, the echo image 21 in the transverse direction of the blood vessel 4 is acquired once again to evaluate the center of the cross section of the blood vessel 4, and from the movement distance of the ultrasound probe 111 and the position of each center, the inclination of the blood vessel 4 in the longitudinal direction is calculated. Based on the calculated inclination of the blood vessel 4 in the longitudinal direction, it is possible to correct the amount of deviation between the guide wire 3 and the center of the blood vessel.

The predetermined technology includes calculating the amount of deviation between the guide wire 3 and the central axis of the blood vessel 4, from the echo image in the transverse direction of the blood vessel 4.

The upper parts illustrated in (1) to (11) of FIGS. 6 to 11 illustrate, in one horizontal row, a schematic view obtained when the subject SU having the blood vessel 4 and the ultrasound probe 111 are viewed from a top surface (from above) of the ultrasound probe 111 in a certain state, a schematic view obtained when the ultrasound probe 111 is viewed from a side surface (from a lateral side) of the ultrasound probe 111, and a schematic view obtained when an ultrasound echo image (the first ultrasound echo image 21 or the second ultrasound echo image 22) obtained at that time.

The lower parts of a series of schematic views illustrated in the upper parts illustrate a schematic view obtained when the subject SU having the blood vessel 4 and the ultrasound probe 111 and the subject SU having the blood vessel 4 and the ultrasound probe 111 when a predetermined operation is applied to the ultrasound probe 111 are viewed from the top surface (from above) of the ultrasound probe 111, a schematic view obtained when the ultrasound probe 111 is viewed from the side surface (from the lateral side), and a schematic view of the ultrasound echo image 20.

In the upper part of FIG. 6(1), the center of the cross section of the blood vessel 4 is evaluated from the echo image 21 of the transverse cross section of the blood vessel 4. In the lower part of FIG. 6(1), the ultrasound probe 111 is rotated around the position at which the ultrasound probe 111 presses the subject SU to detect the echo image 22 passing through the central axis of the blood vessel 4 in the longitudinal direction.

In the upper part of FIG. 6(2), the inclination of the blood vessel 4 in the echo image 22 of the longitudinal cross section passing through the central axis of the blood vessel 4 is evaluated. In the lower part of FIG. 6(2), the ultrasound probe 111 is inclined according to the detected inclination of the blood vessel to detect the echo image 22 of the longitudinal cross section passing through the central axis of the blood vessel 4 and being horizontal to the echo image 20. That is, in the echo image 22, the image of the blood vessel 4 in the longitudinal direction is displayed horizontally.

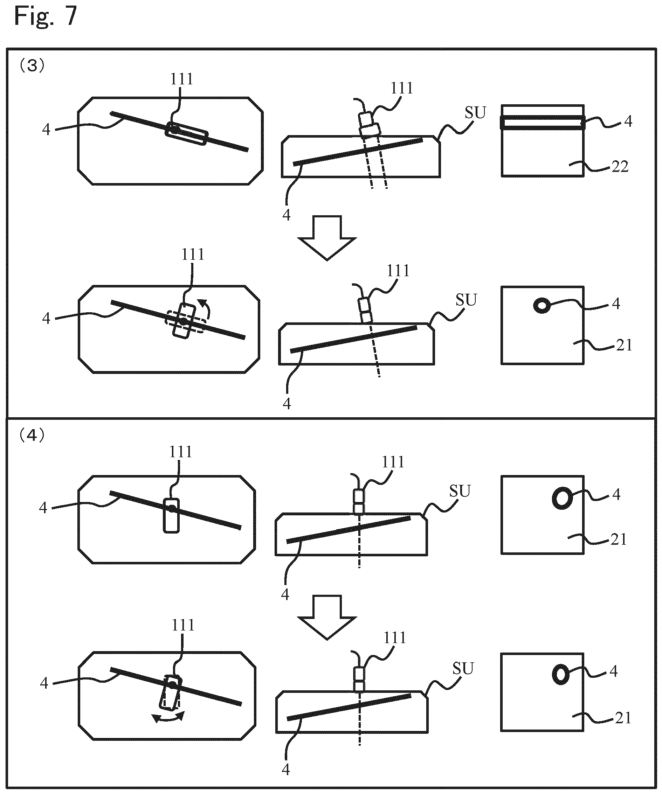

In the upper part of FIG. 7(3), the center of the cross section of the blood vessel 4 is evaluated from the echo image 21 of the transverse cross section of the blood vessel 4 and the ultrasound probe 111 is rotated around the detected position to acquire the echo image 22 of the longitudinal cross section passing through the central axis of the blood vessel to detect the inclination of the blood vessel 4 in the echo image 22. In the lower part of FIG. 7(3), after inclining the ultrasound probe 111 by a predetermined angle according to the detected inclination of the blood vessel 4, the ultrasound probe 111 is rotated by a predetermined angle (for example, 90 degrees) about the Z-axis direction (depth direction of the ultrasound probe) to acquire the transverse cross section 21 of the blood vessel 4.

In the upper part of FIG. 7(4), the center of the cross section of the blood vessel 4 is evaluated from the echo image 21 of the transverse cross section of the blood vessel 4. In the lower part of FIG. 7(4), the ultrasound probe 111 is rotated around the current position at which the ultrasound probe 111 presses against the subject SU to detect a probe position at which the width of the blood vessel cross section in the echo image 21 is minimized.

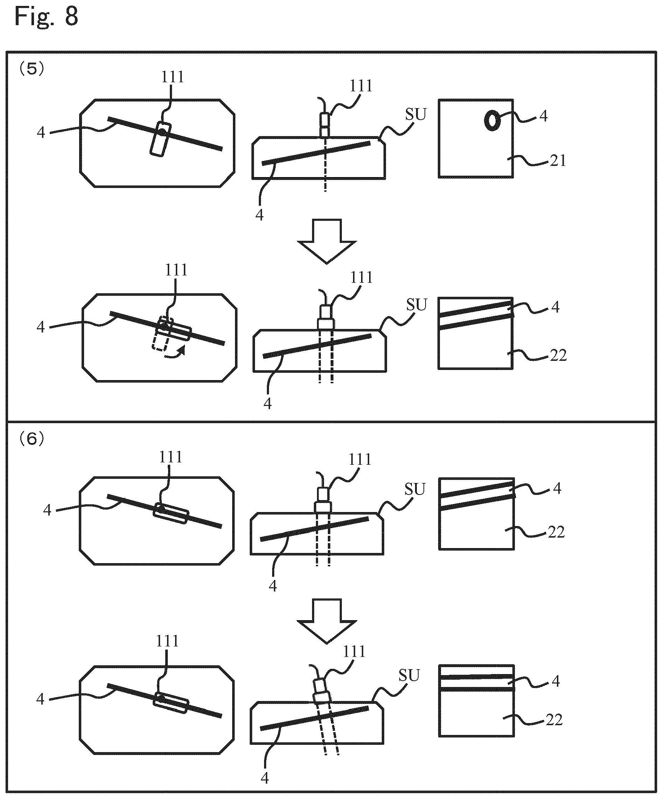

The ultrasound probe 111 is further rotated by a predetermined angle (for example, 90 degrees), as illustrated in the lower part of FIG. 8(5) is rotated from the position at which the width of the blood vessel cross section illustrated in the upper part of FIG. 8(5) is minimized to detect the echo image 22 on the longitudinal cross section of the blood vessel 4.

In the upper part of FIG. 8(6), the inclination in the echo image 22 of the longitudinal cross section passing through the central axis of the blood vessel 4 is evaluated, and in the lower part of FIG. 8(6), the ultrasound probe 111 is inclined according to the inclination of the blood vessel 4 to capture the longitudinal cross section passing through the central axis of the blood vessel 4 and being horizontal in the echo image 22.

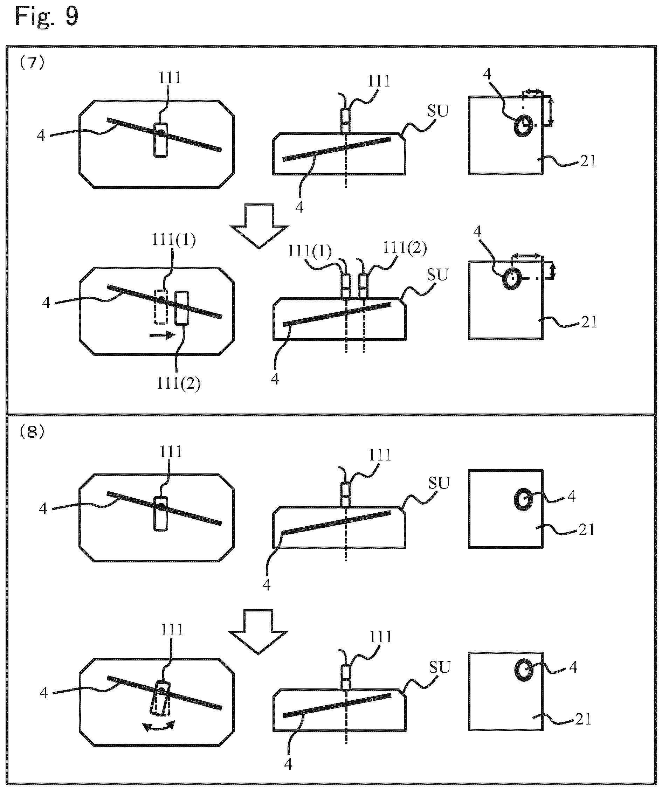

In FIGS. 9 to 11, a plurality of echo images are acquired while moving the ultrasound probe 111 in the longitudinal direction of the blood vessel 4, and the route of the blood vessel 4 is evaluated from the acquired echo image and the position of the ultrasound probe 111.

In FIG. 9(7), the ultrasound probe 111 is moved in the longitudinal direction of the blood vessel, the echo image 21 of the transverse cross section at each position is acquired, and the deviation of the position of the blood vessel 4 in each echo image 21 is calculated.

In FIGS. 9(8) to 11(11), another method is disclosed. In the upper part of FIG. 9(8), the center of the cross section of the blood vessel 4 is evaluated from the echo image 21 of the transverse cross section of the blood vessel 4. In the lower part of FIG. 9(8), the ultrasound probe 111 is rotated around the current position where the ultrasound probe 111 presses against the subject SU to detect a probe position at which the width of the blood vessel cross section in the echo image 21 is minimized.

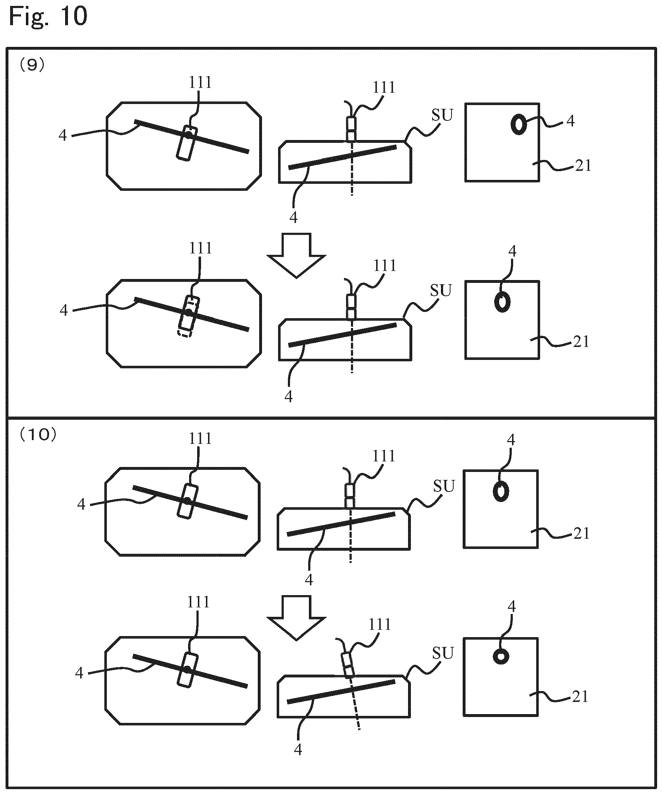

From the upper part of FIG. 10(9) (lower part of FIG. 9(8)), the ultrasound probe 111 is moved in a direction intersecting the longitudinal direction of the blood vessel 4 and the blood vessel 4 is to be displayed at the center in the width direction of the echo image 21.

The ultrasound probe 111 is inclined according to the inclination of the blood vessel 4 from the upper part of FIG. 10(10) (lower part of FIG. 10(9)) to capture the longitudinal cross section passing through the central axis of the blood vessel 4 and being horizontal in the echo image 22.

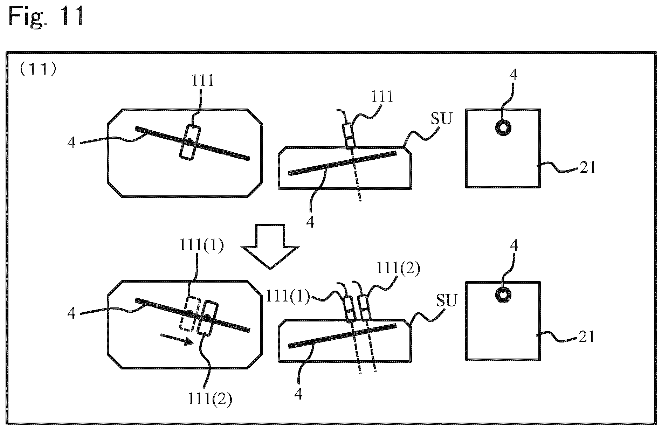

The ultrasound probe 111 is moved in the longitudinal direction of the blood vessel from the upper part of FIG. 11(11) (lower part of FIG. 10(10)), the echo image 21 of the transverse cross section at each position is acquired, and the deviation of the position of the blood vessel 4 in each echo image 21 is calculated.

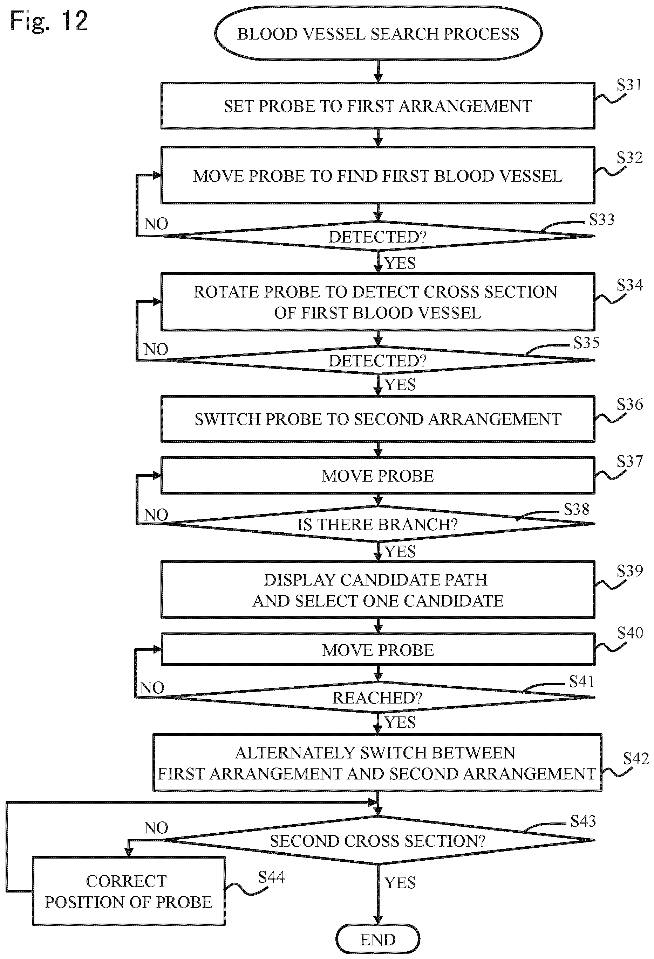

FIG. 12 is a flowchart illustrating a process for searching the blood vessel. Here, a case where a blood vessel in a leg is searched will be described as an example. In such a process, the robot 121 may automatically operate the ultrasound probe 111, or the robot 121 may handle only a part of the operation and the user may handle the other parts of the operation. Here, a case where the robot 121 and the user collaborate to search the blood vessel will be described. In the following description, the robot control device 120 mainly makes a determination, but the user may mainly make a determination. Regardless of whether the robot control device 120 mainly makes a determination or the user makes such a determination, an operation for switching the ultrasound probe 111 between the first arrangement and the second arrangement is executed by the robot control device 120. In FIG. 12, the ultrasound probe is abbreviated as a probe. In the flowchart, to concisely express contents of the steps, the echo image 21 of the transverse cross section of the blood vessel is referred to as "first cross section image 21" and the echo image 22 of the longitudinal cross section is referred to as "second cross section image 22", respectively, but in the following description of the flowchart, for ease of understanding, such cross sections may be referred to as "transverse cross section" and "longitudinal cross section".

The robot control device 120 sets the ultrasound probe 111 to an orientation (first arrangement) being vertical to a body surface and allowing for capturing an image of the transverse cross section of the blood vessel 4 in the vicinity of a groin portion (S31).

The robot control device 120 moves the ultrasound probe 111(S32) until a common femoral artery is detected (S33: NO). When the common femoral artery is detected (S33: YES), the robot control device 120 automatically rotates the orientation of the ultrasound probe 111 to accurately capture an image of the transverse cross section of the common femoral artery (S34).

That is, the robot control device 120 sequentially rotates the orientation of the ultrasound probe 111 to acquire the echo image 21, calculates a score of the echo image 21, and detects a position at which it is possible to accurately capture an image of the transverse cross section of the common femoral artery from the calculated score (S34). The robot control device 120 rotates the ultrasound probe 111 to calculate the score of the transverse cross section until the transverse cross section of the common femoral artery is accurately detected (S35: NO) (S34).

Upon detecting an accurate transverse cross section of the common femoral artery (S35: YES), the robot control device 120 switches the ultrasound probe 111 to the second arrangement to cause the ultrasound probe 111 to capture an image of the cross section in the longitudinal direction of the common femoral artery(S36).

The robot control device 120 moves the ultrasound probe 111 to a peripheral side of the common femoral artery in response to an instruction from the user (S37). The user may instruct the robot control device 120 by voice or a switch operation, for example.

The robot control device 120 determines whether a branch of the common femoral artery is detected (S38). Upon determining that the branch of the common femoral artery is detected (S38: YES), the robot control device 120 displays a candidate path of the ultrasound probe 111 at the branch of the blood vessel on the screen of the user interface device 200 to wait for a selection by the user (S39).

The robot control device 120 moves the ultrasound probe 111 along the path selected by the user (S40) to determine whether to reach an affected area 41(or a predetermined part of the blood vessel to be diagnosed or treated by the long medical device) (S41). Upon determining that the ultrasound probe 111 reaches the affected area 41 (S41: YES), the robot control device 120 alternatively switches the ultrasound probe 111 between the first arrangement and the second arrangement to attempt to detect the cross section in the longitudinal direction passing through the central axis of the common femoral artery (S42).

When the robot control device 120 detects the cross section in the longitudinal direction passing through the central axis of the common femoral artery (S43: YES), the present process ends. If not capable of detecting the cross section in the longitudinal direction passing through the central axis of the common femoral artery (S43: NO), the robot control device 120 corrects the position of the ultrasound probe 111 (S44) and the process returns to step S43.

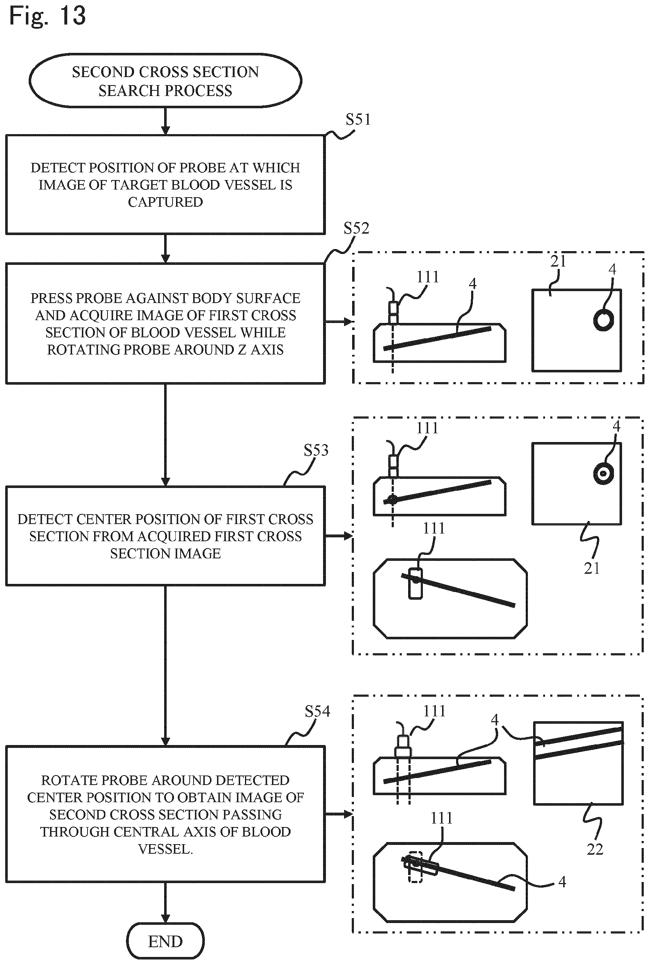

FIG. 13 is a flowchart illustrating a process for searching the cross section in the longitudinal direction of a blood vessel ahead of the blood vessel under observation. Such a process can be used, for example, in steps S36 and S42 described in FIG. 12. It is noted that FIGS. 13 to 15 schematically illustrate a relationship between the position of the ultrasound probe 111 and the image for some steps.

The robot control device 120 detects a position of the probe at which it is possible to capture an image of a target blood vessel (S51), presses the ultrasound probe 111 against the body surface at the detected position, and rotates the ultrasound probe 111 in the Z-axis direction to acquire the echo image 21 (first cross section image 21) of the transverse cross section of the target blood vessel (hereinafter, blood vessel) (S52).

The robot control device 120 detects the center position of the cross section of the blood vessel from the plurality of first cross section images 21 acquired in step S52 (S53).

The robot control device 120 rotates the ultrasound probe 111 by using the center position detected in step S53 as the rotation center to obtain the echo image 22 (second cross section image 22) of the longitudinal cross section passing through the central axis of the blood vessel (S54).

According to the present embodiment configured thus, when the ultrasound probe 111 is switched between the first arrangement and the second arrangement, it is possible to accurately and easily detect the position of the blood vessel to improve the user-friendliness.

In the present embodiment, the user and the robot 121 (and the robot control device 120) cooperate to operate the ultrasound probe 111, and thus, for example, while the user is allowed to make a high level determination such as whether the path at the branch of the blood vessel is selected or whether the ultrasound probe 111 reaches the affected area 41, the robot is allowed to perform an accurate operation such as switching between the first arrangement and the second arrangement with the center point CP of the distal end lens of the ultrasound probe 111 being fixed. Therefore, according to the present embodiment, it is possible to easily search the blood vessel by the ultrasound probe 111 while reducing a burden of the user.

### [Second Embodiment]

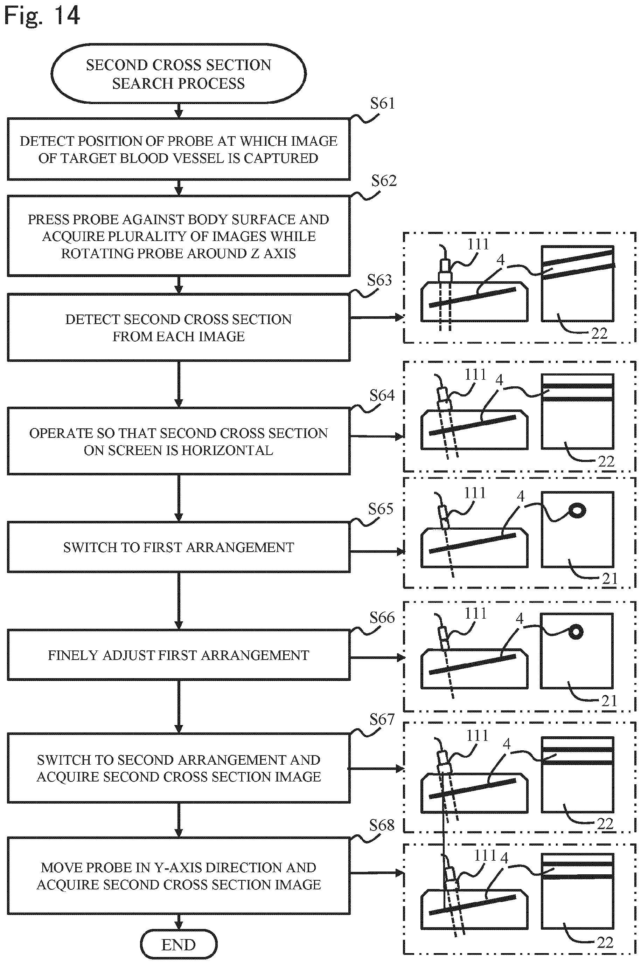

With reference to FIG. 14, a second embodiment will be described. In each of the following embodiments including the present embodiment, a difference from the first embodiment will be mainly described.

FIG. 14 is a flowchart illustrating a process for searching the cross section in the longitudinal direction of a blood vessel ahead of the blood vessel under observation.

The robot control device 120 detects a position of the probe at which it is possible to capture an image of a target blood vessel (S61), presses the ultrasound probe 111 against the body surface at the detected position, and rotates the ultrasound probe 111 in the Z-axis direction to acquire the echo image 21 of the cross section of the blood vessel at each position (S62).

The robot control device 120 selects the echo image 22 of the longitudinal cross section of the blood vessel, from the plurality of echo images acquired in step S62, based on the length of the cross section of the blood vessel along a width direction W of the echo image (S63).

The robot control device 120 pivots (inclines) the ultrasound probe 111 around the X-axis direction while fixing the center point CP of the distal end lens of the ultrasound probe 111 to set the second cross section image of the blood vessel to be horizontal in the echo image 22 (S64).

The robot control device 120 rotates the ultrasound probe 111 around the Z-axis direction by 90 degrees while fixing the center point CP of the distal end lens of the ultrasound probe 111 to set the ultrasound probe 111 to the first arrangement and acquires the echo image 21 (first cross section image 21) of the transverse cross section of the blood vessel (S65).

The robot control device 120 pivots the ultrasound probe 111 around the X-axis direction while fixing the center point CP of the distal end lens of the ultrasound probe 111 to obtain an accurate first cross section image 21 (S66). That is, the robot control device 120 finely adjusts the position of the ultrasound probe 111 to acquire the correct first cross section image 21.

The robot control device 120 rotates the ultrasound probe 111 around the Z-axis direction by 90 degrees to set the ultrasound probe 111 to the second arrangement while fixing the center point CP of the distal end lens of the ultrasound probe 111, and acquires the second cross section image 22 being the longitudinal cross section of the blood vessel (S67).

The robot control device 120 moves the ultrasound probe 111 in the Y-axis direction to further acquire the second cross section image of the blood vessel (S68).

The present embodiment configured thus also exhibits a similar operation and effect to that of the first embodiment.

### [Third Embodiment]

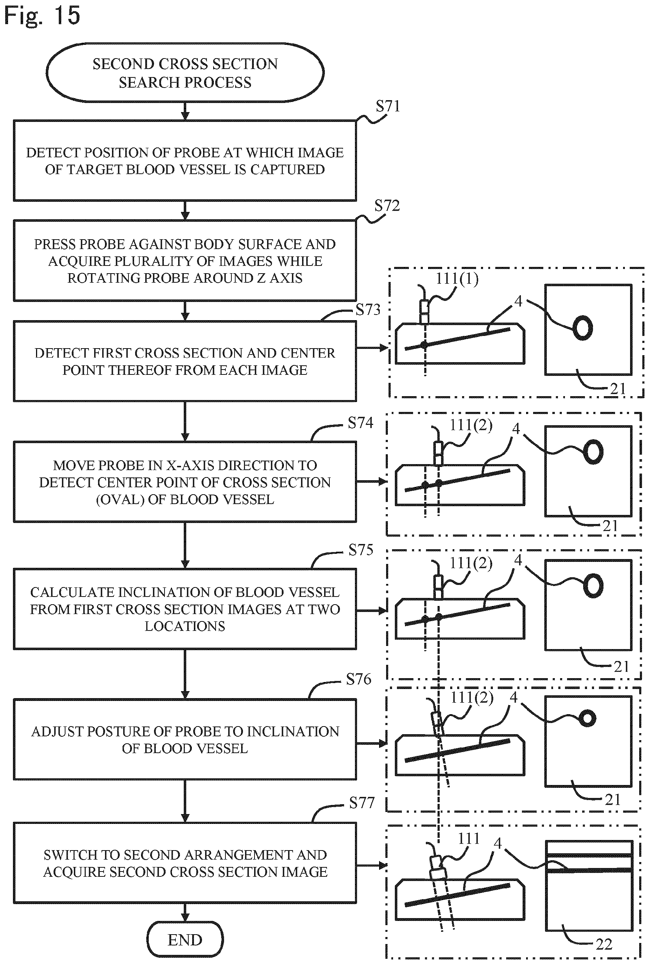

With reference to FIG. 15, a third embodiment will be described. FIG. 15 is a flowchart illustrating a process for searching the cross section in the longitudinal direction of a blood vessel ahead of the blood vessel under observation.

The robot control device 120 detects a position of the probe at which it is possible to capture an image of a target blood vessel (S71), presses the ultrasound probe 111 against the body surface at the detected position, and rotates the ultrasound probe 111 in the Z-axis direction to acquire the echo image 21 of the cross section of the blood vessel at each position (S72).

The robot control device 120 selects the echo image 21 (first cross section image) of the transverse cross section of the blood vessel, from the plurality of echo images acquired in step S72, based on the length of the cross section of the blood vessel along the width direction WL of the echo image, and detects the center point of the selected first cross section image (S73). Examples of the method of selecting one echo image from the plurality of echo images include a method of selecting the first cross section image having the longest length of the cross section of the blood vessel along the width direction WL, a method of selecting the first cross section image having the largest area value, and a method of selecting the first cross section image in consideration of both the length of the cross section of the blood vessel and the area of the cross section of the blood vessel.

The robot control device 120 moves the ultrasound probe 111 in the X-axis direction to detect the center point of another cross section of the blood vessel (S74). The cross sections of the blood vessel detected in steps S73 and S74 are elliptical.

The robot control device 120 calculates the inclination of the blood vessel from the positions of the center points of the two cross sections of the blood vessel and the movement distance of the ultrasound probe 111 (S75).

The robot control device 120 adjusts the posture of the ultrasound probe 111 to the inclination of the blood vessel while fixing the center point CP of the distal end lens of the ultrasound probe 111 (S76).

The robot control device 120 rotates the ultrasound probe 111 around the Z-axis direction by 90 degrees to set the ultrasound probe 111 to the second arrangement while fixing the center point CP of the distal end lens of the ultrasound probe 111, and acquires the echo image 22 of the longitudinal cross section of the blood vessel (S75).

The present embodiment configured thus also exhibits a similar operation and effect to that of the first embodiment.

### [Fourth Embodiment]

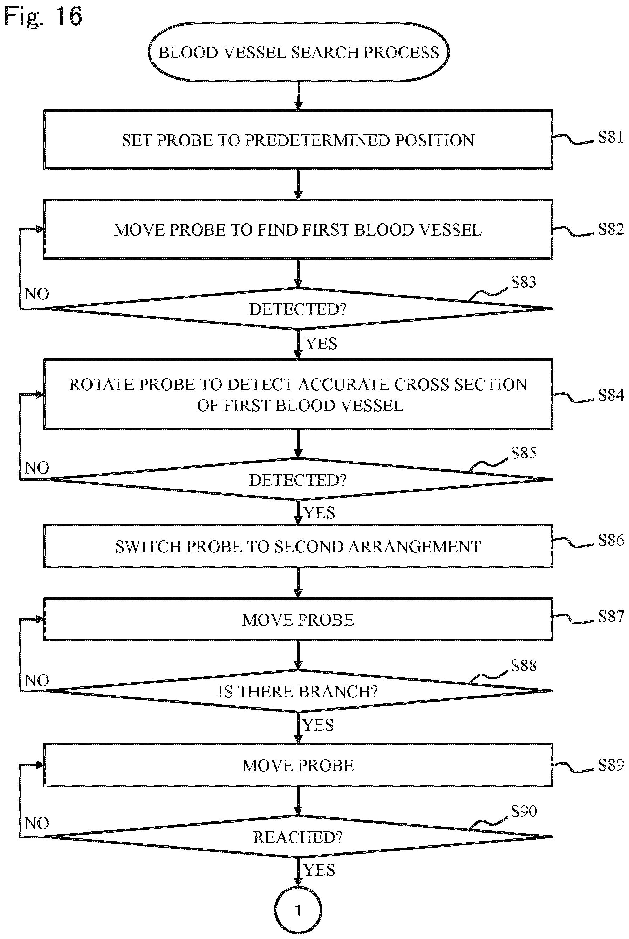

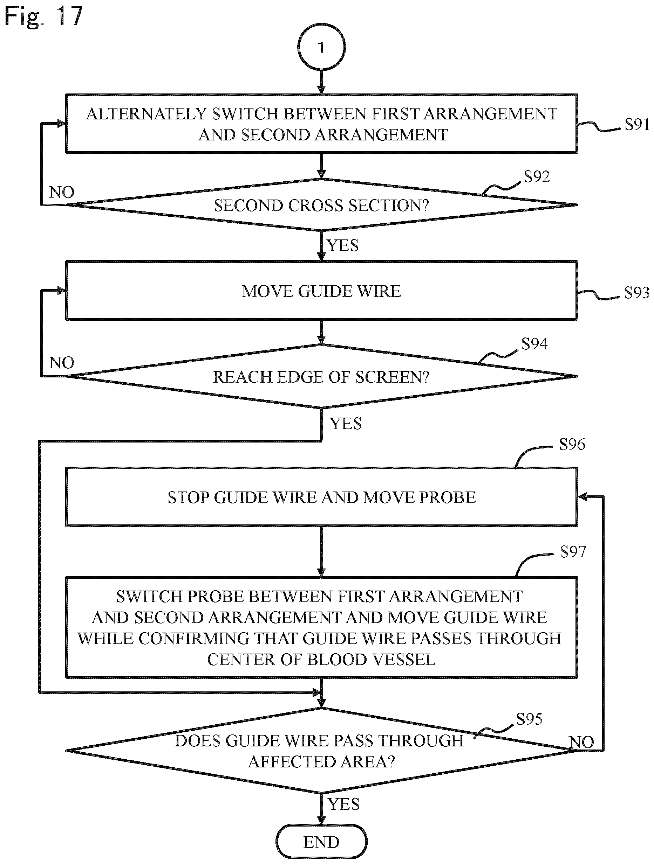

With reference to FIGS. 16 and 17, a fourth embodiment will be described. In the present embodiment, an operation of the ultrasound probe 111 and an operation of the guide wire 3 will be described. In the present embodiment, an operator as a first user operates the ultrasound probe 111, and a doctor as a second user instructs the operator to operate the ultrasound probe 111. The doctor also operates the guide wire 3. Determination of the transverse cross section and the vertical cross section of the blood vessel is assisted by the robot control device 120.

The operator places the ultrasound probe 111 at a predetermined position on the body surface of the subject SU (S81). The predetermined position here is, for example, a position in the vicinity of the groin portion, and a position at which it is possible to capture an image of the transverse cross section of the blood vessel relative to the body surface.

The operator moves the ultrasound probe 111 so that an image of a first blood vessel being a target blood vessel is captured by the ultrasound probe 111 (S82). The first blood vessel is the common femoral artery, for example.

The operator moves the ultrasound probe 111 on the body surface until an image of the femoral artery as the first blood vessel is captured by the ultrasound probe 111 (S83: NO) (S82). Upon an image of the femoral artery being captured by the ultrasound probe 111 (S83: YES), the operator rotates the orientation of the ultrasound probe 111 to correctly detect the transverse cross section of the femoral artery (S84). It is possible to obtain a plurality of echo images by sequentially rotating the orientation of the ultrasound probe 111, and the score of each echo image is automatically calculated. Here, the score is an index for knowing whether the transverse cross section is close to an exact transverse cross section, and is displayed on the screen of the user interface device 200. The operator changes the orientation of the ultrasound probe 111 with reference to the score to accurately capture the transverse cross section of the common femoral artery (S84).

When the transverse cross section of the common femoral artery is accurately captured (S85: YES), the operator selects a function of switching the ultrasound probe 111 from the first arrangement to the second arrangement, and captures an image of the longitudinal cross section of the common femoral artery (S86). An instruction of the operator is transmitted to the robot control device 120 by a voice operation, a switch operation, or the like.

The operator moves the ultrasound probe 111 to the peripheral side of the common femoral artery and searches the blood vessel until the branch of the common femoral artery is found (S87, S88). When the branch of the common femoral artery is detected (S88: YES), the operator moves the ultrasound probe 111 to the peripheral side along a femoral artery of the branch of the common femoral artery near the body surface (S89).

When the ultrasound probe 111 reaches the affected area 41 (S90: YES), the operator refers to the score of each echo image obtained by sequentially rotating the orientation of the ultrasound probe 111 to detect the transverse cross section of the common femoral artery, and once the transverse cross section is accurately detected, the robot control device 120 is instructed to set the ultrasound probe 111 to the second arrangement (S91).

When the longitudinal cross section of the common femoral artery is detected by the ultrasound probe 111 set to the second arrangement (S92: YES), the operator switches the ultrasound probe 111 to the first arrangement at any time according to the doctor's instruction to confirm that the guide wire 3 passes through the center of the blood vessel on the transverse cross section, and the doctor advances the guide wire 3 to the end of the image 22 on the longitudinal cross section (S93).

When the guide wire 3 advances to the end of the screen 22 (S94: YES), the doctor determines whether the guide wire 3 passes the terminal end of the affected area 41 (S95). When the guide wire 3 passes the terminal end of the affected area 41 (S95: YES), the present process ends.

If the guide wire 3 does not pass the terminal end of the affected area 41 (S95: NO), the doctor stops feeding the guide wire 3 and orders the operator to move the ultrasound probe 111 to the peripheral side of the common femoral artery (S96).

The operator switches the ultrasound probe 111 to the first arrangement at any time according to the doctor's instruction to confirm that the guide wire 3 passes through the center of the blood vessel on the transverse cross section, and the doctor advances the guide wire 3 to the end of the image 22 on the longitudinal cross section (S97).

The present embodiment configured thus also exhibits a similar operation and effect to that of the first embodiment.

### [Fifth Embodiment]

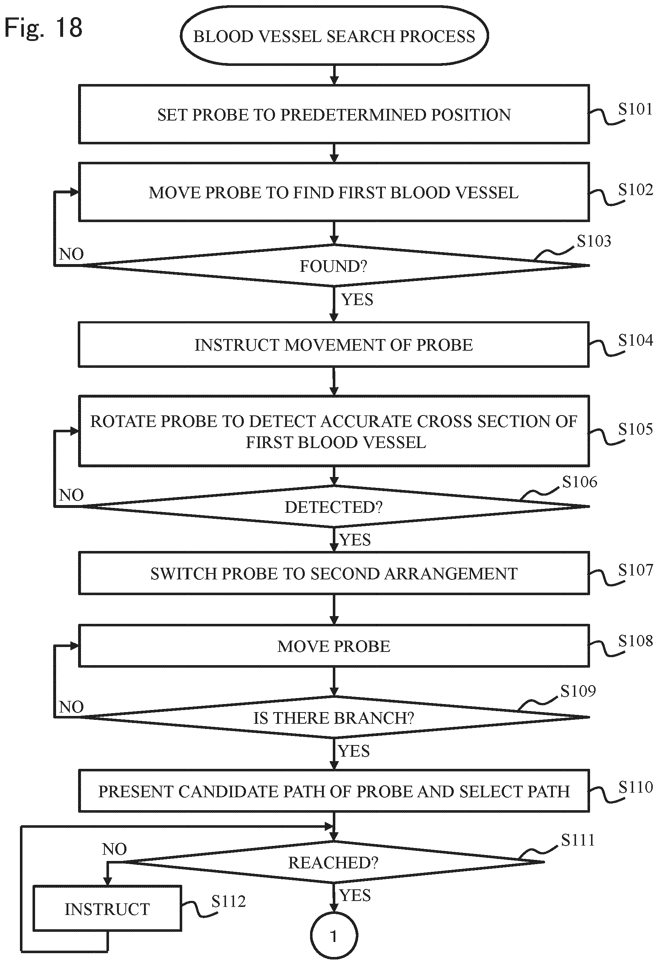

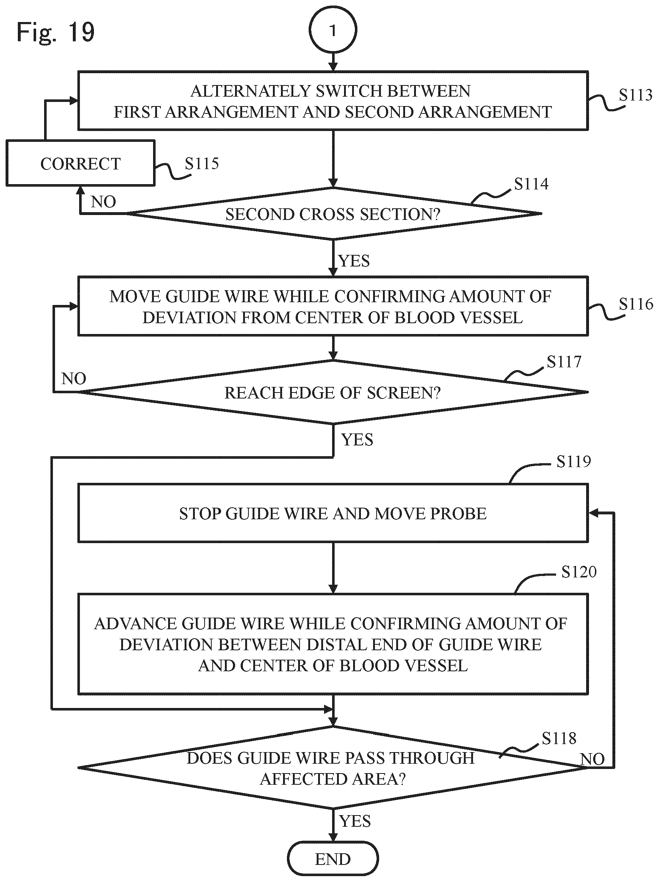

With reference to FIGS. 18 and 19, a fifth embodiment will be described. In the present embodiment, the robot control device 120 operates the ultrasound probe 111 and a doctor instructs the robot control device 120 to operate the ultrasound probe 111. The doctor also operates the guide wire 3. Determination of the transverse cross section and the vertical cross section of the blood vessel is assisted by the robot control device 120.

The robot control device 120 places the ultrasound probe 111 at a predetermined position on the body surface of the subject SU (S101).

The robot control device 120 moves the ultrasound probe 111 so that an image of the first blood vessel (femoral artery) being a target blood vessel is captured by the ultrasound probe 111 (S102).

The robot control device 120 moves the ultrasound probe 111 on the body surface until an image of the femoral artery as the first blood vessel is captured by the ultrasound probe 111 (S103: NO) (S102). When the robot control device 120 detects the femoral artery by the ultrasound probe 111 (S103: YES), the doctor instructs the robot control device 120 to capture an image of the transverse cross section (S104).

The robot control device 120 that receives the instruction by the doctor rotates the orientation of the ultrasound probe 111 to correctly detect the transverse cross section of the femoral artery (S105).

The robot control device 120 acquires the plurality of echo images by sequentially rotating the orientation of the ultrasound probe 111, changes the orientation of the ultrasound probe 111, based on the score of each echo image, and exactly captures the transverse cross section of the common femoral artery (S105).

The robot control device 120 asks the doctor to confirm whether an image of the transverse cross section of the common femoral artery is accurately captured. Upon confirmation by the doctor (S106: YES), the robot control device 120 switches the ultrasound probe 111 from the first arrangement to the second arrangement to capture an image of the longitudinal cross section of the common femoral artery (S107). It is possible to execute a request from the robot control device 120 to the doctor via the user interface device 200.

The robot control device 120 moves the ultrasound probe 111 to the peripheral side of the common femoral artery according to the instruction from the doctor (S108). The doctor moves the ultrasound probe 111 until the branch of the common femoral artery is found (S109: NO).

When the branch of the common femoral artery is detected (S109: YES), the robot control device 120 displays a candidate path of the ultrasound probe 111 at the detected branch on the screen of the user interface device 200 (S110). The doctor selects the path to move the ultrasound probe 111 to the peripheral side along the femoral artery of the branch of the common femoral artery near the body surface (S110).

Until the ultrasound probe 111 reaches the affected area 41 (S111: NO), the doctor issues a movement instruction to the robot control device 120 (S112).

The robot control device 120 switches the ultrasound probe 111 alternately between the first arrangement and the second arrangement, captures an image of the longitudinal cross section (second cross section) passing through the central axis of the femoral artery, and displays such a cross section on the user interface device 200 (S113).

Until the ultrasound probe 111 accurately captures the longitudinal cross section (S114: NO), the position of the ultrasound probe 111 is corrected (S115).

When the ultrasound probe 111 accurately captures the longitudinal cross section (S114: YES), the robot control device 120 moves the ultrasound probe 111 according to the doctor's instruction and displays the transverse cross section of the femoral artery at the distal end of the guide wire 3 (S116). In step S116, the amount of deviation between the position of the distal end of the guide wire 3 and the center of the blood vessel of the femoral artery is calculated, and such an amount is provided, together with the transverse cross section image, to the doctor. The doctor advances the guide wire 3 to the end of the image 22 of the longitudinal cross section while confirming the amount of deviation (S116).

When the guide wire 3 advances to the end of the screen 22 (S117: YES), the doctor determines whether the guide wire 3 passes the terminal end of the affected area 41 (S118). When the guide wire 3 passes the terminal end of the affected area 41 (S118: YES), the present process ends.

If the guide wire 3 does not pass the terminal end of the affected area 41 (S118: NO), the doctor stops feeding the guide wire 3 and orders the robot control device 120 to move the ultrasound probe 111 to the peripheral side of the common femoral artery (S119).

Similarly to the description in step S116, the robot control device 120 displays the first cross section image of the femoral artery, and the amount of deviation between the center of the blood vessel of the femoral artery and the distal end of the guide wire 3, on the user interface device 200. The doctor advances the guide wire 3 toward the affected area 41 while confirming such a display (S120).

The present embodiment configured thus also exhibits a similar operation and effect to that of the first embodiment.

### [Sixth Embodiment]

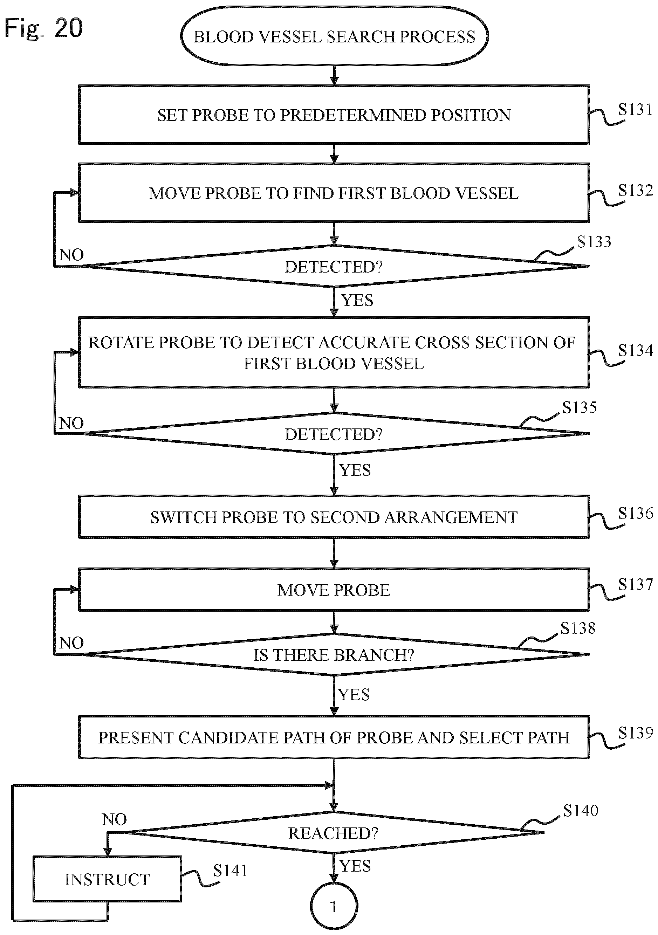

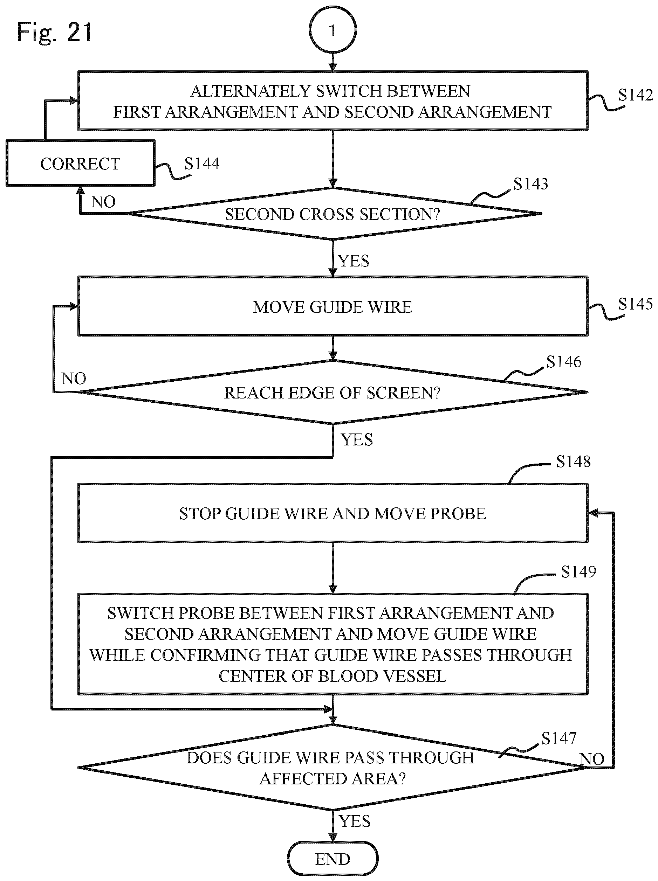

With reference to FIGS. 20 and 21, a sixth embodiment will be described. In the present embodiment, the robot control device 120 operates the ultrasound probe 111, and the robot control device 120 operates the ultrasound probe 111 under the confirmation of the doctor. The doctor operates the guide wire 3.

The robot control device 120 places the ultrasound probe 111 at a predetermined position on the body surface of the subject SU (S131).

The robot control device 120 moves the ultrasound probe 111 so that an image of the first blood vessel (femoral artery) being a target blood vessel is captured by the ultrasound probe 111 (S132).

The robot control device 120 moves the ultrasound probe 111 (S132) until an image of the femoral artery as the first blood vessel is captured by the ultrasound probe 111 (S133: NO).

When the robot control device 120 detects the femoral artery by the ultrasound probe 111 (S133: YES), the robot control device 120 rotates the orientation of the ultrasound probe 111 to correctly detect the transverse cross section of the femoral artery (S134). The robot control device 120 acquires the plurality of echo images by sequentially rotating the orientation of the ultrasound probe 111, changes the orientation of the ultrasound probe 111, based on the score of each echo image, and exactly captures the transverse cross section of the common femoral artery (S134).

The robot control device 120 asks the doctor to confirm whether an image of the transverse cross section of the common femoral artery is accurately captured. Upon confirmation by the doctor (S135: YES), the robot control device 120 switches the ultrasound probe from the first arrangement to the second arrangement to capture an image of the longitudinal cross section of the common femoral artery (S136).

The robot control device 120 moves the ultrasound probe 111 to the peripheral side of the common femoral artery according to the instruction from the doctor (S137). The doctor moves the ultrasound probe 111 until the branch of the common femoral artery is found (S138: NO).

When the branch of the common femoral artery is detected (S138: YES), the robot control device 120 displays a candidate path of the ultrasound probe 111 at the detected branch on the screen of the user interface device 200 (S139). The doctor selects the path to move the ultrasound probe 111 to the peripheral side along the femoral artery of the branch of the common femoral artery near the body surface (S139).

Until the ultrasound probe 111 reaches the affected area 41 (S140: NO), the doctor issues a movement instruction to the robot control device 120 (S141).

The robot control device 120 switches the ultrasound probe 111 alternately between the first arrangement and the second arrangement, captures an image of the longitudinal cross section (second cross section) passing through the central axis of the femoral artery to display such a cross section on the user interface device 200 (S142).

Until the ultrasound probe 111 accurately captures the longitudinal cross section (S143: NO), the position of the ultrasound probe 111 is corrected (S144).

When the ultrasound probe 111 accurately captures the longitudinal cross section (S143: YES), the robot control device 120 moves the ultrasound probe 111 and displays the transverse cross section of the femoral artery at the distal end of the guide wire 3 (S145). In step S145, the amount of deviation between the position of the distal end of the guide wire 3 and the center of the blood vessel of the femoral artery is calculated, and such an amount is provided, together with the transverse cross section image, to the doctor. The doctor advances the guide wire 3 to the end of the image 22 of the longitudinal cross section while confirming the amount of deviation (S145).

When the guide wire 3 advances to the end of the screen 22 (S146: YES), the doctor determines whether the guide wire 3 passes the terminal end of the affected area 41 (S147). When the guide wire 3 passes the terminal end of the affected area 41 (S147: YES), the present process ends.

If the guide wire 3 does not pass the terminal end of the affected area 41 (S147: NO), the doctor stops feeding the guide wire 3 and orders the robot control device 120 to move the ultrasound probe 111 to the peripheral side of the common femoral artery (S148).

The robot control device 120 displays the first cross section image of the femoral artery, and the amount of deviation between the center of the blood vessel of the femoral artery and the distal end of the guide wire 3, on the user interface device 200. The doctor advances the guide wire 3 toward the affected area 41 while confirming such a display (S149).

The present embodiment configured thus also exhibits a similar operation and effect to that of the first embodiment.

### [Seventh Embodiment]

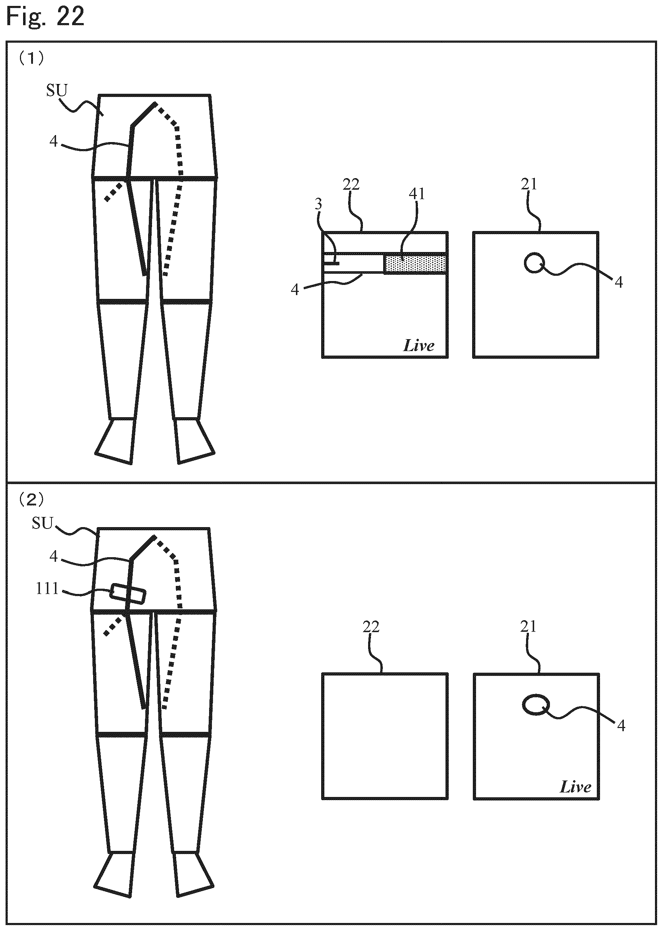

With reference to FIGS. 22 to 27, a seventh embodiment will be described. In the present embodiment, description proceeds for an example of a relationship between the operation of the ultrasound probe 111 and each screen 21 and 22.

In the present embodiment, as described above, each of the plurality of echo images obtained by the ultrasound probe 111 is displayed on a separate screen.

The other one of the plurality of echo images is the image 21 of the transverse cross section for determining the amount of deviation of the distal end of the guide wire from the center of the blood vessel. The image 21 of the transverse cross section is a cross section image passing through the center in the transverse direction (short axis direction) of the blood vessel. One of the plurality of echo images is the image 22 of the longitudinal cross section for moving the distal end of the guide wire 3 in the blood vessel, for example. The image 22 of the longitudinal cross section is a cross section image passing through the center in the longitudinal direction (long axis direction) of the blood vessel.

In the present embodiment, as described above, from among the plurality of echo images, the echo image corresponding to the current position of the ultrasound probe 111 is distinguished from another echo image. A word "Live" is used in the present embodiment as a marker for the distinction.

In the present embodiment, the Live image may be switched from among the plurality of echo images according to the operation of the ultrasound probe 111 or the guide wire 3. As a result, even if the position of the ultrasound probe 111 is moved, it is possible to return the ultrasound probe 111 to the original position, and thus, when the ultrasound probe 111 is rotated or moved, it is possible to instantly switch the Live image between the "image 21 of the transverse cross section" and the "image 22 of the longitudinal cross section".

Further, even if the doctor or the operator takes his or her eyes off the monitor screen of the user interface device 200 or the position of the ultrasound probe 111, it is possible to easily reproduce the Live image, and thus, no inconvenience occurs, and therefore, it is easier for doctors and the like to confirm a state of a patient (subject SU).

FIG. 22(1) illustrates a basic example of each image 21 and 22. In FIG. 22(2), the ultrasound probe 111 is oriented perpendicularly to the body surface to draw a transverse cross section of the blood vessel, in the vicinity of the groin portion.

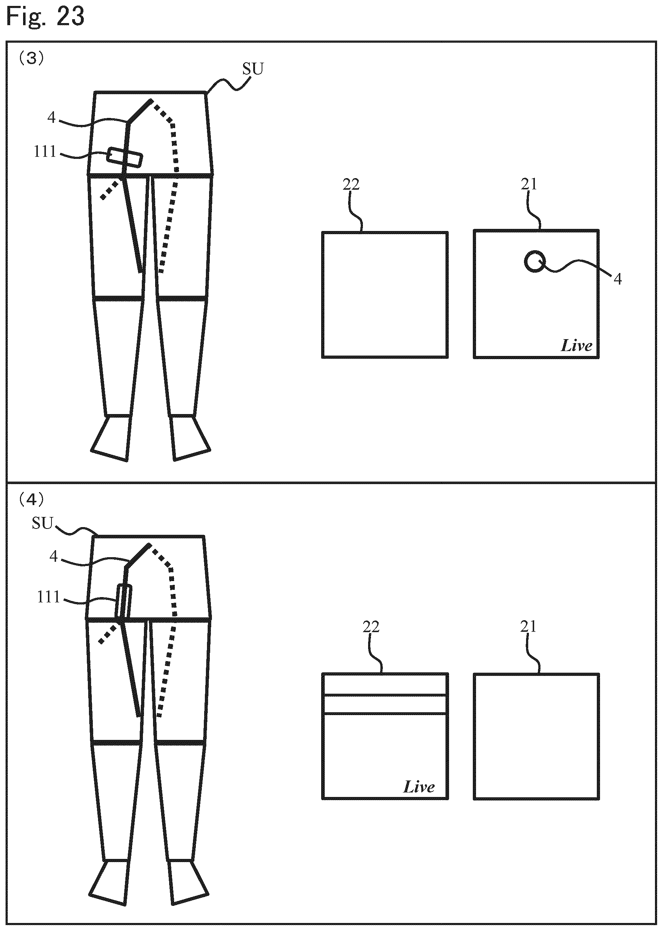

In FIG. 23(3), when the common femoral artery is detected, the orientation of the ultrasound probe 111 is rotated to accurately capture the transverse cross section of the artery.

In FIG. 23(4), the ultrasound probe 111 is rotated by 90 degrees to capture an image of the longitudinal cross section of the common femoral artery.

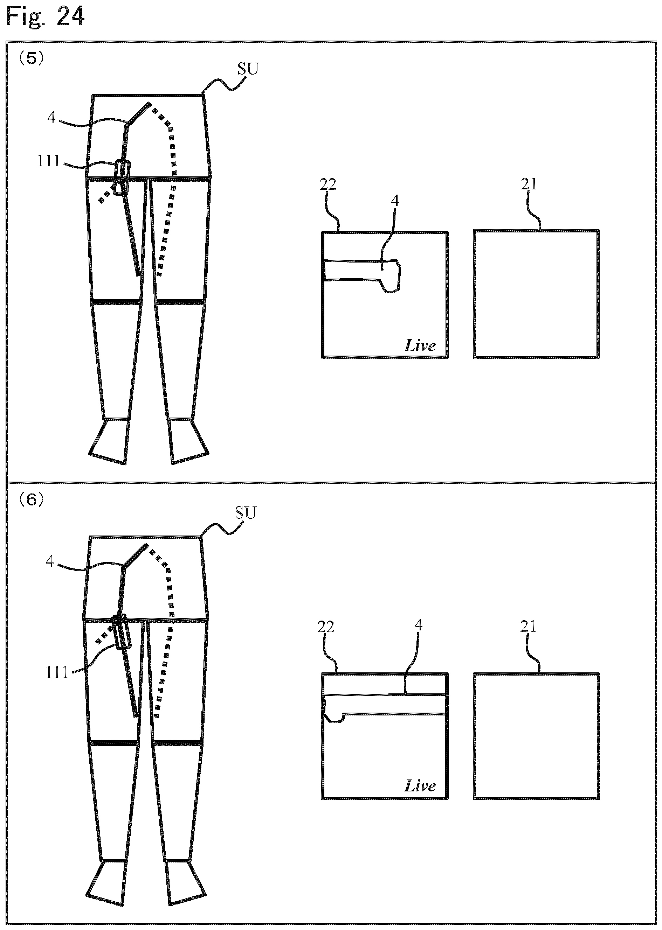

In FIG. 24(5), the ultrasound probe 111 is moved to the peripheral side of the common femoral artery to search the branch of the common femoral artery.

In FIG. 24(6), the ultrasound probe 111 is advanced to the peripheral side along a superficial femoral artery on a shallow side of the branch of the common femoral artery near the body surface.

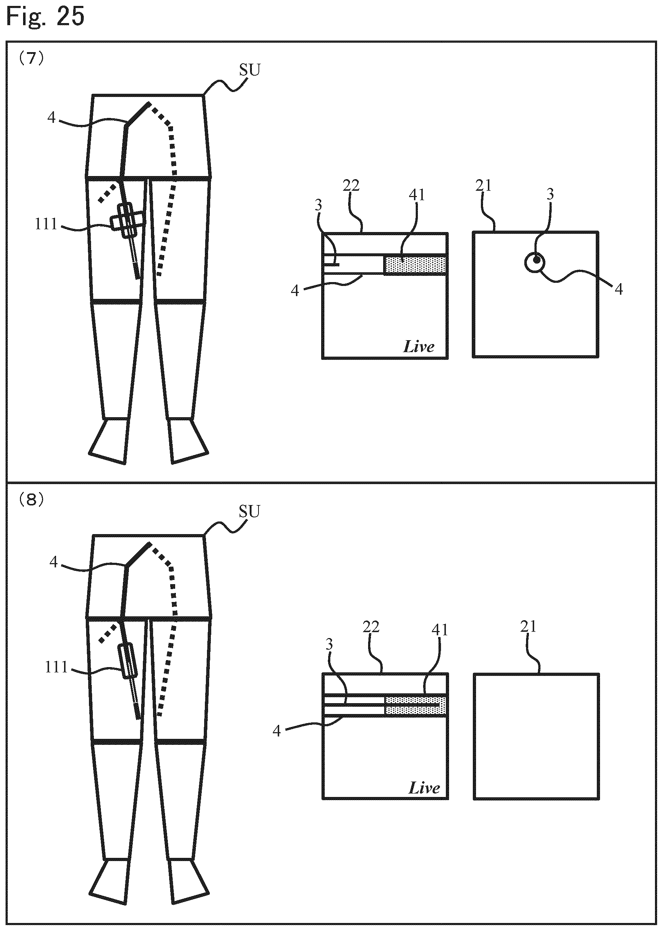

In FIG. 25(7), when the ultrasound probe 111 reaches the affected area, the longitudinal cross section passing through the central axis of the artery is captured while rotating the ultrasound probe 111 and alternately confirming the transverse cross section and the longitudinal cross section.

In FIG. 25(8), the guide wire 3 is advanced to the end of the screen while the longitudinal cross section passing through the central axis of the artery is fixed.

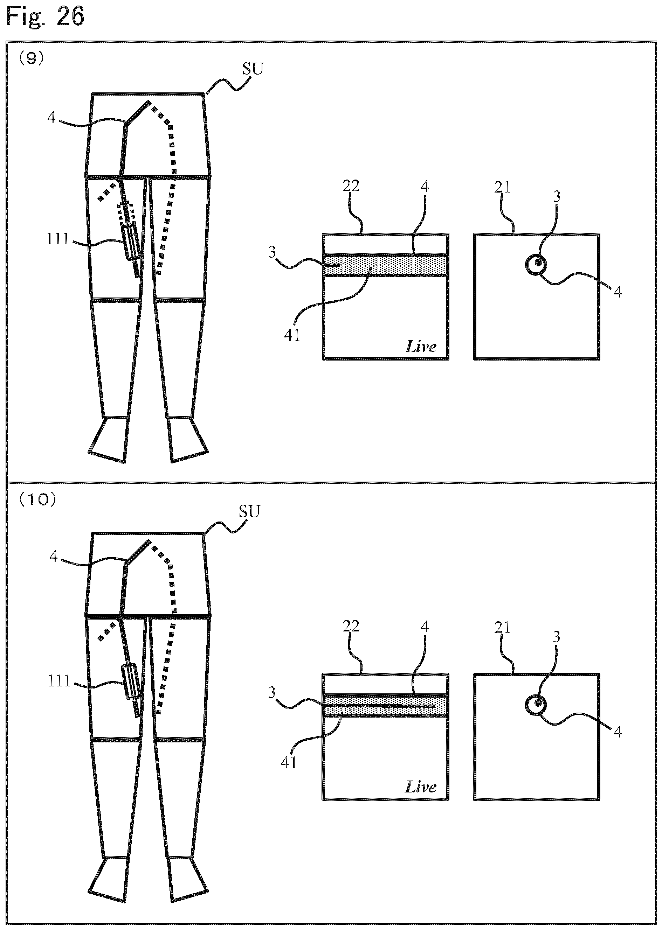

In FIG. 26(9), the feeding of the guide wire 3 is stopped, and the ultrasound probe 111 is moved to the peripheral side of the blood vessel.

In FIG. 26(10), the guide wire 3 is advanced toward the end of the screen while the longitudinal cross section passing through the central axis of the artery is fixed again. Similarly below, FIGS. 26(9) and 26(10) are repeated until the ultrasound probe 111 reaches the affected area.

It is noted that the disclosed embodiments are not limited to the above-described embodiments, and include various modifications. The above-described embodiments are described in detail to facilitate understanding of the disclosed embodiments, and are not necessarily limited to an embodiment including all the described configurations. A part of the configuration of one embodiment may be replaced with a configuration of another embodiment. A configuration of another embodiment may be added to a configuration of a certain embodiment. A part of the configuration of each embodiment may be added with, deleted by, and replaced with another configuration.

Each constituent component of the disclosed embodiments may be arbitrarily selected, and the invention including the selected configuration is also included in the disclosed embodiments. Further, the configurations described in the claims may be combined in addition to the combinations specified in the claims. Each of the above-mentioned embodiments may be arbitrarily combined.

It is noted that the guide wire is described as an example, but the disclosed embodiments are not limited to an object called the guide wire at the time of the present application. The disclosed embodiments can be applied to an object that is inserted into a tube and moves therein, the object needing to be detected in position.

In the above description, a predetermined technology is described in which the center of the cross section of the blood vessel is evaluated from the echo image in the transverse direction of the blood vessel and the amount of deviation of the long medical device is evaluated from the central axis of the blood vessel. The disclosed embodiments may be applied to a technology in which the center of the cross section of the blood vessel is evaluated from the echo image of the blood vessel in the longitudinal direction and the amount of deviation of the long medical device from the central axis of the blood vessel is evaluated. The disclosed embodiments may be applied to a technology in which an inner wall surface (inner edge) of the blood vessel is evaluated from the echo image in the transverse direction (or longitudinal direction) of the blood vessel and the amount of deviation of the long medical device is evaluated from the inner wall surface (inner edge) of the blood vessel.

### DESCRIPTION OF REFERENCE NUMERALS

1 Control portion
2 Display portion
3 Guide wire
4 Blood vessel
11 First arrangement portion
12 Second arrangement portion
13 Ultrasound echo image processing portion
14 Computation portion
15 Warning portion
16 Blood vessel inclination calculation portion
17 Deviation amount calculation portion
18 Blood vessel route detection portion
19 Ultrasound probe posture control portion
21 First ultrasound echo image (image of transverse cross section of blood vessel)
22 Second ultrasound echo image 22 (image of longitudinal cross section of blood vessel)
110 Ultrasound diagnostic device
120 Robot control portion
200 User interface device

## Claims

1. An ultrasound probe operation system for operating an ultrasound probe, comprising:
a sensor operation portion that operates an ultrasound probe that acquires an ultrasound echo image of a blood vessel; and
a control portion that controls an operation of the sensor operation portion, wherein
the control portion switches an arrangement of the ultrasound probe using the sensor operation portion between a first arrangement for acquiring an image of a cross section in a transverse direction of the blood vessel and a second arrangement for acquiring an image of a cross section in a longitudinal direction of the blood vessel.

2. The ultrasound probe operation system according to claim 1, wherein the operation portion switches the arrangement between the second arrangement and the first arrangement by rotating the ultrasound probe around a predetermined rotation axis.

3. The ultrasound probe operation system according to claim 2, further comprising: a display portion that displays the ultrasound echo image acquired by the ultrasound probe, wherein
the control portion displays a first ultrasound echo image acquired in the first arrangement and a second ultrasound echo image acquired in the second arrangement in an associated manner on the display portion.

4. The ultrasound probe operation system according to claim 3, wherein the control portion displays, on the display portion, an ultrasound echo image corresponding to a current or past arrangement of the ultrasound probe, separately from an ultrasound echo image corresponding to an arrangement other than the current or past arrangement, out of the first ultrasound echo image and the second ultrasound echo image.

5. The ultrasound probe operation system according to claim 3, wherein the ultrasound echo image includes an image of a long medical device in the blood vessel, and
the control portion calculates an amount of deviation between the long medical device and the blood vessel in the first ultrasound echo image and/or the second ultrasound echo image.

6. The ultrasound probe operation system according to claim 5, wherein the control portion displays the calculated amount of deviation on the display portion in association with the first ultrasound echo image and/or the second ultrasound echo image.

7. The ultrasound probe operation system according to claim 5, wherein the control portion outputs an alarm when the amount of deviation reaches a predetermined threshold value.

8. The ultrasound probe operation system according to claim 3, wherein the control portion detects center positions of the blood vessel in the first ultrasound echo image at a plurality of locations separated in the longitudinal direction of the blood vessel, and based on a distance between the center positions and a distance between the plurality of locations, calculates an inclination of the blood vessel in the longitudinal direction.

9. The ultrasound probe operation system according to claim 5, wherein the control portion sets the ultrasound probe to the first arrangement at a plurality of locations separated in the longitudinal direction of the blood vessel to detect center positions of the blood vessel in the first ultrasound echo image, and based on a distance between the center positions and a distance between the plurality of locations, calculates an inclination of the blood vessel in the longitudinal direction to correct the amount of deviation, based on the calculated inclination.

10. The ultrasound probe operation system according to claim 8, wherein the control portion acquires the second ultrasound echo image as a longitudinal cross section image passing through each of the center positions of the blood vessel, based on the calculated inclination of the blood vessel in the longitudinal direction.

11. The ultrasound probe operation system according to claim 10, wherein the control portion controls a posture of the ultrasound probe according to the calculated inclination of the blood vessel in the longitudinal direction.

12. The ultrasound probe operation system according to claim 11, wherein the control portion presses the ultrasound probe against a body surface by inclining the ultrasound probe according to the calculated inclination of the blood vessel in the longitudinal direction to set the ultrasound probe to the second arrangement so that an image of the blood vessel in the longitudinal direction is displayed horizontally in the second ultrasound echo image.

13. The ultrasound probe operation system according to claim 12, wherein the control portion switches the arrangement between the first arrangement and the second arrangement by rotating, by a predetermined angle, the inclined ultrasound probe being pressed against the body surface.

14. The ultrasound probe operation system according to claim 3, wherein the control portion detects center positions of the blood vessel in the first ultrasound echo image at a plurality of locations separated in the longitudinal direction of the blood vessel, and based on a distance between the center positions and a distance between the plurality of locations, detects a route of the blood vessel.

15. A method of operating an ultrasound probe by a robot, comprising:
switching, by the robot, an arrangement of the ultrasound probe that acquires an ultrasound echo image of a blood vessel between a first arrangement for acquiring an image of a cross section in a transverse direction of the blood vessel and a second arrangement for acquiring an image of a cross section in a longitudinal direction of the blood vessel.

16. The method of operating an ultrasound probe according to claim 15, wherein the robot switches the arrangement between the first arrangement and the second arrangement by rotating the ultrasound probe around a predetermined rotation axis.

17. The method of operating an ultrasound probe according to claim 16, further comprising: displaying an ultrasound echo image corresponding to a current or past arrangement of the ultrasound probe, separately from an ultrasound echo image corresponding to an arrangement other than the current or past arrangement, out of a first ultrasound echo image acquired in the first arrangement and a second ultrasound echo image acquired in the second arrangement.

18. The method of operating an ultrasound probe according to claim 16, wherein the ultrasound echo image includes an image of a long medical device in the blood vessel, and
in the first ultrasound echo image, an amount of deviation between the long medical device and the blood vessel is calculated.

19. The method of operating an ultrasound probe according to claim 17, further comprising: detecting center positions of the blood vessel in the first ultrasound echo image at a plurality of locations separated in the longitudinal direction of the blood vessel; and
calculating an inclination of the blood vessel in the longitudinal direction, based on a distance between the center positions and a distance between the plurality of locations.

20. The method of operating an ultrasound probe according to claim 19, further comprising: acquiring the second ultrasound echo image as a longitudinal cross section image passing through each of the center positions of the blood vessel, based on the calculated inclination of the blood vessel in the longitudinal direction.
